(19) Europäisches Patentamt
European Patent Office
Office européen des brevets

(11) **EP 2 014 635 A1**

(12) **EUROPEAN PATENT APPLICATION**

(43) Date of publication:
**14.01.2009 Bulletin 2009/03**

(21) Application number: **07252370.7**

(22) Date of filing: **12.06.2007**

(51) Int Cl.:
*C07C 6/10* (2006.01)     *C07C 9/10* (2006.01)
*C07C 9/14* (2006.01)     *C10G 50/00* (2006.01)
*C07C 2/76* (2006.01)     *C07C 9/06* (2006.01)

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(71) Applicant: **BP OIL INTERNATIONAL LIMITED Sunbury-on-Thames TW16 7BP (GB)**

(72) Inventor: **The designation of the inventor has not yet been filed**

(74) Representative: **De Kezel, Eric et al BP International Limited Patents & Agreements Chertsey Road Sunbury-on-Thames TW16 7LN (GB)**

(54) **Process for converting ethane into liquid alkane mixtures**

(57) The invention relates to a process for converting ethane into liquid mixture of ($C_4$+) alkanes having 4 carbon atoms and more, preferably ($C_{5+}$) alkanes having 5 carbon atoms and more. The process comprises a stage (1) comprising simultaneous ethane self- and cross-metathesis reactions carried out by contacting ethane with a metal catalyst (C1) capable of producing, in contact with alkane, reactions involving the splitting and recombining of C-C and/or C-H and/or C-metal bonds, so as to form a reaction mixture (M1) comprising methane and a mixture of the ($C_{4+}$), preferably the ($C_{5+}$) alkanes, a stage (2) comprising separating and isolating methane from the reaction mixture (M1), a stage (3) comprising a non-oxidative methane coupling reaction carried out by contacting the methane thus isolated with a metal catalyst (C2) capable of producing, in contact with alkane, reactions involving the splitting and recombining of C-C and/or C-H and/or C-metal bonds, said catalyst (C2) being identical to or different from the catalyst (C1), so as to form a mixture (M2) comprising ethane and hydrogen, optionally a stage (4) comprising separating and isolating ethane from the mixture (M2), a stage (5) comprising recycling the ethane thus isolated or the mixture (M2) into stage (1) for going on with the simultaneous ethane self- and cross-metathesis reactions and continuing forming said reaction mixture (M1), and a stage (6) comprising separating and isolating the mixture of the (C4+), preferably the (C5+) alkanes in a liquid form, stage (6) being preferably performed in combination and particularly simultaneously with stage (2).

EP 2 014 635 A1

**Description**

The present invention relates to a process for converting ethane into liquid alkane mixtures.

Ethane is generally present in relatively great amounts in natural gas and represents the second major component of the natural gas after methane. As natural gas is very widely distributed in the world, and its deposit sites are often very far removed from one another and from industrial areas where ethane can be collected and used, it follows that often ethane present in natural gas has to be transported over long distances. It is however difficult to transport ethane as well as natural gas because of their gaseous nature. Both can nevertheless be liquefied and transported more easily in liquid form. However, the energy and capital costs in achieving the liquefaction conditions and in transporting ethane or natural gas in liquid form are very high and may be often prohibitive as regards the exploitation of ethane and natural gas deposits, particularly ones of low capacity.

A solution would be to convert ethane into higher alkane mixtures which would be liquid under standard temperature and pressure conditions (or conditions close to the latter), i.e. into liquid alkane mixtures, in particular liquid mixtures of ($C_{4+}$) alkanes, i.e. alkanes having 4 carbon atoms and more, (e.g. liquid mixtures of $C_4$ to $C_{15}$ alkanes), or preferably liquid mixtures of ($C_{5+}$) alkanes, i.e. alkanes having 5 carbon atoms and more, (e.g. liquid mixtures of $C_5$ to $C_{15}$ alkanes), and which could therefore be easily transportable in liquid form. Such a conversion process would have to be simple and realisable at relatively low costs, enabling it in particular to be installed directly on the natural gas deposit sites. This is the solution which the present invention proposes.

International Patent Application WO 98/02244 discloses a process for converting alkanes into their higher and lower homologues in the presence of a metal catalyst suitable for alkane metathesis and chosen in particular from metal hydrides and organometallic compounds fixed to a solid support. The Application proposes two methods. A first method comprises reacting a linear, branched or cyclic substituted hydrocarbon with itself, so as to obtain its higher and lower homologues by a self-metathesis reaction according to equation (1):

$$C_nH_{2n+2} \rightarrow C_{n-i}H_{2(n-i)+2} + C_{n+i}H_{2(n+i)+2} \qquad (1)$$

wherein i = 1, 2, 3 ... n-1. All the examples of the Application relate to this first method, and show the self-metathesis reactions successively of ethane, propane, butane and isobutane. In particular, Example 3 shows a self-metathesis of ethane producing methane and propane as the predominant final products, with traces of isobutane and n-butane, i.e. essentially a gaseous alkane mixture. However, Example 3 neither discloses, nor suggests how to produce liquid alkane mixtures, in particular liquid mixtures of ($C_{4+}$), preferably ($C_{5+}$) alkanes. The Application also discloses a second method comprising reacting between themselves at least two hydrocarbons selected from linear, branched and cyclic substituted hydrocarbons, so as to obtain the branched or cyclic substituted hydrocarbons. However, no more information relating to this method is given. The Application teaches that in any case, higher hydrocarbons are always produced simultaneously with lower hydrocarbons which unavoidably lead to increase in the gaseous nature of the reaction mixtures. The Application does not suggest a method for preparing liquid alkanes mixtures in particular from ethane, without producing the undesirable lighter, normally gaseous alkanes.

International Patent Applications WO 01/04077 and WO 03/066552 disclose processes for preparing alkanes by reacting methane with at least one other starting alkane in the presence of an alkane metathesis metal catalyst capable of producing, in contact with alkane, reactions involving the splitting and recombining of carbon-carbon and/or carbon-hydrogen and/or carbon-metal bonds, in particular a metal catalyst chosen from the catalysts mentioned in International Patent Application WO 98/02244. Said reaction, known under the term "methane-olysis" permits the preparation of at least one or two alkanes having a number of carbon atoms less than or equal to that of the initial alkane and at least equal to 2. Various methane-olysis reactions are shown as examples, in particular by reacting methane with ethane, propane or n-butane. It appears that methane-olysis of ethane leads to formation of methane and ethane, that is to say to formation of the starting products of the reaction. In addition, it is shown that methane-olysis of propane leads to formation of ethane, and that of n-butane permits ethane and propane to be prepared. However, the methane-olysis process involves the utilisation of methane necessarily with another starting alkane, and leads to the formation of alkanes whose number of carbon atoms is not higher than that of the starting alkane. No liquid alkane mixture is shown to be prepared in particular from ethane.

International Patent Application WO 03/104171 discloses a process for converting methane into ethane by contacting methane with an alkane metathesis metal catalyst chosen in particular from the catalysts mentioned in International Patent Application WO 98/02244. The process employs a methane coupling reaction, also called a methane homologation reaction. It is stated that the process forms ethane which can then be used in other processes for upgrading of the ethane, in particular processes selected from dehydrogenation, catalytic cracking and thermal cracking, in order to prepare more particularly olefins, such as ethylene. International Patent Application WO 03/104171 does not envisage a process for preparing liquid alkane mixtures in particular from ethane.

International Patent Application WO 2006/060692 discloses supported metal clusters comprising one or more metals of Groups 4, 5 and/or 6 of the Periodic Table of the Elements, and which can be used as catalysts for alkane reactions. More particularly, the supported metal clusters are capable of being used to catalyze the metathesis (also referred to as disproportionation) of alkanes, conversions of alkanes with each other to give alkane products with molecular weights different from those of the starting alkanes, and conversion of methane to higher molecular-weight alkanes. The only alkane reactions described and shown as examples relate to a self-metathesis of ethane to give propane and methane, and a conversion of methane and n-butane (i.e. a methane-olysis of n-butane) to give propane and ethane. However, the Application neither discloses, nor suggests a process for converting ethane into liquid alkane mixtures in particular from ethane. Indeed, it is shown that methane reacting with other starting alkane (e.g. n-butane) leads to formation of final alkanes (e.g. propane and ethane) with molecular weight lower than that of the starting alkane.

Thus, it appears that none of the above-mentioned patent applications proposes a process for converting ethane into liquid alkane mixtures, in particular into liquid mixtures of ($C_{4+}$) alkanes, i.e. alkanes having 4 carbon atoms and more, (e.g. liquid mixtures of $C_4$ to $C_{15}$, or $C_4$ to $C_{13}$, or $C_4$ to $C_{10}$, or $C_4$ to $C_8$ alkanes), preferably liquid mixtures of ($C_{5+}$) alkanes, i.e. alkanes having 5 carbon atoms and more, (e.g. liquid mixtures of $C_5$ to $C_{15}$, or $C_5$ to $C_{13}$, or $C_5$ to $C_{10}$, or $C_5$ to $C_8$ alkanes), and capable more particularly of being easily transportable in liquid form, e.g. under standard temperature and pressure conditions (or conditions close to the latter).

A process has now been found for converting ethane into liquid alkane mixtures, in particular liquid mixtures of ($C_{4+}$), preferably ($C_{5+}$) alkanes, such as the above-mentioned liquid mixtures, and capable in particular of being easily transportable in liquid form, e.g. under standard temperature and pressure conditions (or conditions close to the latter). The process has the advantage of being able to produce liquid alkane mixtures, in particular liquid mixtures of ($C_{4+}$), preferably ($C_{5+}$) alkanes, such as the above-mentioned liquid mixtures, having a high degree of purity and in particular being free of alkenes, alkynes, aromatic compounds, carbon monoxide, carbon dioxide, sulfur- and/or nitrogen-containing products. It also has the advantage of being able to produce said liquid mixtures of alkanes with a narrow distribution of the number of carbon atoms, in particular ranging for example from 4 to 15 or preferably from 5 to 15, particularly from 4 to 13 or preferably from 5 to 13, more particularly from 4 to 10 or preferably from 5 to 10, or even from 4 to 8 or preferably from 5 to 8 carbon atoms. The present invention has, in addition, the advantage of proposing a process by a non-oxidising method, of being simple and direct, and consequently of being relatively inexpensive, so that in particular it can be directly used on the natural gas deposit sites where ethane is present in relatively great amounts. Furthermore, one of the most important advantages of the process of the present invention relates to its potential for an extremely high "carbon atom efficiency" which can approach 95% or higher for converting ethane into easily transportable liquid mixtures of higher alkanes, such as the above-mentioned liquid alkane mixtures.

More particularly, the present invention relates to a process for converting ethane into a liquid mixture of ($C_{4+}$) alkanes having 4 carbon atoms and more, preferably ($C_{5+}$) alkanes having 5 carbon atoms and more, characterised in that it comprises:

- a stage (1) comprising simultaneous ethane self- and cross-metathesis reactions carried out by contacting ethane with a metal catalyst (C1) capable of producing, in contact with alkane, reactions involving the splitting and recombining of carbon-carbon and/or carbon-hydrogen and/or carbon-metal bonds, so as to form a reaction mixture (M1) comprising methane and a mixture of the ($C_{4+}$), preferably the ($C_{5+}$) alkanes,
- a stage (2) comprising separating methane from the reaction mixture (M1), so as to isolate the methane,
- a stage (3) comprising a non-oxidative methane coupling reaction carried out by contacting the methane isolated in stage (2) with a metal catalyst (C2) capable of producing, in contact with alkane, reactions involving the splitting and recombining of carbon-carbon and/or carbon-hydrogen and/or carbon-metal bonds, said catalyst being identical to or different from the catalyst (C1), so as to form a mixture (M2) comprising ethane and hydrogen,
- optionally a stage (4) comprising separating ethane from the mixture (M2), so as to isolate the ethane,
- a stage (5) comprising recycling the ethane thus isolated or the mixture (M2) into stage (1) for simultaneous ethane self- and cross-metathesis reactions and for continuing forming the reaction mixture (M1), and
- a stage (6) comprising separating the mixture of the ($C_{4+}$), preferably the ($C_{5+}$) alkanes from the reaction mixture (M1), so as to isolate the liquid mixture of the ($C_{4+}$), preferably the ($C_{5+}$) alkanes, stage (6) being preferably performed in combination with stage (2).

The ethane used in stage (1) can be any ethane, in particular selected from ethane separated from natural gas, or from ethane resulting from a process manufacturing ethane as the main product or as a by-product. The most important source of ethane is natural gas which can be any natural gas, such that exploited directly on a deposit site, and generally formed of mixtures of alkanes comprising mainly methane and, in weaker proportions, ethane and ($C_{3+}$) alkanes, i.e. alkanes having at least 3 carbon atoms, especially $C_3$ to $C_8$, particularly $C_3$ to $C_7$, especially $C_3$ to $C_6$ alkanes, e.g. propane, butanes, pentanes and hexanes, and also sometimes traces of heptane(s) and higher alkane(s).

In natural gas, ethane and the ($C_{3+}$) alkanes generally are present with the following percentages expressed in mole:

- ethane from 1 to 20 % , preferably from 2 to 15 % , particularly from 3 to 12 % ,
- propane from 0.1 to 10 % , preferably from 0.1 to 6 % , particularly from 0.1 to 4 % ,
- butanes (n-butane and isobutane) from 0 to 3 % , preferably from 0.01 to 2 %, particularly from 0.01 to 1 % ,
- pentanes (n-pentane and isopentane) from 0 to 2 % , preferably from 0.01 to 1 % , and
- hexane(s) from 0 to 1 %, preferably from 0.01 to 0.1%.

In the contacting of stage (1), ethane can be used alone or substantially alone, i.e. in the form of a mixture of ethane with 1% by mole or less of one or more other alkane(s) preferably selected from methane and ($C_{3+}$) alkanes having at least 3 carbon atoms, e.g. $C_3$ to $C_8$, more particularly $C_3$ to $C_7$, preferably $C_3$ to $C_6$ alkanes, such as the alkanes of the natural gas from which ethane can be derived. Ethane can be also used in the form of a mixture of ethane, preferably in a major molar proportion, with one or more other alkane(s) preferably selected from ($C_{3+}$) alkanes having at least 3 carbon atoms, e.g. linear or branched alkanes, preferably $C_3$ to $C_8$, more particularly $C_3$ to $C_7$, especially $C_3$ to $C_6$ alkanes, such as the ($C_{3+}$) alkanes of the natural gas from which ethane can be issued. More particularly, ethane can be used in the form of a mixture comprising more than 50% and less than 99%, preferably from 60 to less than 99% of moles of ethane.

More particularly, ethane can be used in stage (1) in the form of a mixture with one or more ($C_{3+}$) alkanes of the natural gas, preferably the $C_3$ to $C_8$, particularly the $C_3$ to $C_7$, especially the $C_3$ to $C_6$ alkanes of the natural gas, in particular in minor molar proportions preferably identical or lower than to those existing in the natural gas between the ($C_{3+}$) alkanes and the ethane. Thus, prior to stage (1), ethane can be separated from natural gas in a preliminary stage comprising fractionating the natural gas into ethane optionally in a mixture with one or more ($C_{3+}$) alkanes of the natural gas, preferably the $C_3$ to $C_8$, particularly the $C_3$ to $C_7$, especially the $C_3$ to $C_6$ alkanes of the natural gas, preferably in the above-mentioned molar proportions, so as to isolate the ethane optionally in said mixture with one or more ($C_{3+}$) alkanes.

Ethane which can be derived from natural gas, may comprise nitrogen ($N_2$) and one or more poisons generally present in natural gas and which are capable of deactivating metal catalysts. Thus, the present process may comprise, prior to stage (1), a purification stage comprising removing from the ethane or the natural gas from which ethane can be issued, one or more poison(s) capable of deactivating in particular the metal catalyst(s) (C1) and/or (C2) employed in the present process, and optionally nitrogen ($N_2$). The purification stage can comprise more particularly removing one or more poison(s) chosen preferably from water, carbon dioxide and sulfur compounds especially selected from hydrogen sulfide, carbonyl sulfide and mercaptans, and optionally nitrogen ($N_2$). Any method can be used for removing nitrogen ($N_2$) and these poisons.

Several natural gas deposits may comprise substantial concentrations of nitrogen ($N_2$) as impurity. Since nitrogen ($N_2$) is not anticipated to harm the metal catalysts (C1) and/or (C2), it might not be necessary to remove the nitrogen ($N_2$) from ethane or the natural gas from which ethane can be issued, prior to contacting ethane with these catalysts in the present process, in particular if the concentration of nitrogen is small, e.g. lower than 1% by volume of the ethane. However, it is advisable to remove nitrogen ($N_2$) from ethane or from the natural gas from which ethane can be derived, before contacting ethane with these catalysts in the present process.

Water can also be removed by subjecting ethane or the natural gas from which ethane can be derived, to a dehydration process, preferably chosen from cryogenic dehydration, dehydration by absorption and adsorptive dehydration. A cryogenic dehydration generally comprises cooling the wet ethane or the wet natural gas until the components to be removed precipitate by condensation or formation of hydrates. Methanol, glycol or a paraffin solvent can be used during such a cryogenic dehydration. The reduction in temperature can be achieve e.g. by Joule-Thomson expansion.

Dehydration by absorption generally comprises a glycol absorption, wherein glycol such as mono-, di- or preferably triethylene glycol exhibits a high absorption capacity for water.

Adsorptive dehydration generally comprises contacting ethane or the natural gas from which ethane can be derived, with any adsorbent suitable for retaining water, preferably chosen from molecular sieves, silica gels and $Na_2O$-containing silica.

Sulfur compounds and carbon dioxide can be also removed from ethane or the natural gas from which ethane can be derived, by any process preferably chosen from physical absorption processes, chemical absorption processes, physical-chemical absorption processes, liquid oxidation processes, adsorption processes and membrane processes.

Generally, absorption processes comprise a gas scrubbing wherein the sour ethane or the sour natural gas components are reversibly bound to a solvent by chemical and/or physical absorption. In regeneration step, the components are then desorbed unchanged and the solvent can be recycled to the scrubber. In physical absorption processes, carbon dioxide and hydrogen sulfide are generally physically dissolved in a solvent preferably chosen from N-methylpyrrolidone, a mixture of poly(ethylene glycol dimethyl ether), poly(ethylene glycol methyl isopropyl ether) and propylene carbonate. Chemical absorption processes generally comprise an alkanolamine scrubbing wherein aqueous solutions of monoethanolamine, diethanolamine, diisopropylamine, diglycolamine or methyldiethanolamine can be used as absorbents. Physical-chemical absorption processes generally comprise using a combination of solvents described previously in physical absorption and chemical absorption processes.

Liquid oxidation processes generally comprise the following steps: (i) absorption of hydrogen sulfide in an alkaline solution, e.g. a solution of $NaHCO_3$-$Na_2CO_3$, (ii) oxidation of dissolved hydrogen sulfide ions preferably by vanadates or iron(III) aminopolycarboxylic acid chelates, to elemental sulfur with simultaneously reduction of a chemical oxygen carrier, e.g. anthraquinonedisulfonic acids, (iii) reoxidation of the active component with air, and (iv) separation of elemental sulfur by filtration, centrifugation, flotation or sedimentation.

Adsorption processes for removal of hydrogen sulfide and carbon dioxide generally comprise contacting ethane or the natural gas from which ethane can be derived, with any adsorbent preferably chosen from activated charcoal, iron oxide, zinc oxide and zeolitic molecular sieves.

Membrane processes especially used for natural gas can be simultaneously performed for separation of carbon dioxide and methane, removal of carbon dioxide and hydrogen sulfide, dehydration and recovery of ethane and the ($C_{3+}$) alkanes, preferably the $C_3$ to $C_8$, particularly the $C_3$ to $C_7$, especially the $C_3$ to $C_6$ alkanes. Generally, membrane processes comprise the following steps: (i) absorption from the gas phase into the membrane matrix, (ii) diffusion through the membrane, and (iii) desorption out the membrane into the gas phase. Membranes generally are polymer membranes preferably chosen from asymmetric cellulose acetate or triacetate, composite layers of silicone/polysulfone, composite layers polyetherimide and composite layers of silicone/polycarbonate. Any membrane process may be used to remove the nitrogen ($N_2$) from ethane or the natural gas from which ethane can be issued. Some membranes are capable of separating more than one component, and therefore depending on the composition of the mixture comprising ethane or the natural gas from which ethane can be issued, appropriate separation methods have to be chosen.

Preferably, ethane used in stage (1) can be previously separated from natural gas in a preliminary stage comprising fractionating natural gas into (a) methane, (b) ethane and (c) a mixture of the ($C_{3+}$) alkanes (having at least 3 carbon atoms), preferably the $C_3$ to $C_8$, particularly the $C_3$ to $C_7$, especially the $C_3$ to $C_6$ alkanes of the natural gas (e.g. propane, butanes, pentanes and hexanes), so as to separate and to isolate the ethane. The preliminary stage may also comprise fractionating natural gas into (a) methane and (b) ethane in a mixture with the mixture of the ($C_{3+}$) alkanes, preferably the $C_3$ to $C_8$, particularly the $C_3$ to $C_7$, especially the $C_3$ to $C_6$ alkanes of the natural gas, so as to separate and to isolate the ethane in a mixture with said mixture of the ($C_{3+}$) alkanes, preferably the $C_3$ to $C_8$, particularly the $C_3$ to $C_7$, especially the $C_3$ to $C_6$ alkanes. More particularly, the preliminary stage can be performed in various ways, either intermittently (or discontinuously), or preferably continuously. It can comprise one or more methods of fractionation of the natural gas, chosen from any method or combination of methods for fractionation of the natural gas, in particular for fractionation between (a) methane, (b) ethane and (c) a mixture of the ($C_{3+}$) alkanes of the natural gas, or between (a) methane and (b) ethane in a mixture with the mixture of the ($C_{3+}$) alkanes of the natural gas. The methods of fractionation are preferably chosen from the following six types of fractionation:

- fractionation by change of physical state, preferably of gaseous/liquid phase of the natural gas, in particular by distillation or by condensation (or liquefaction), more particularly by partial condensation (or partial liquefaction), in particular by means of distillation/condensation column or tower,
- fractionation by molecular filtration, preferably by means of semi-permeable and selective membrane,
- fractionation by adsorption, preferably by means of molecular sieve or any other adsorbent,
- fractionation by absorption, in particular by means of absorbing oil,
- fractionation by cryogenic expansion, in particular by means of expansion turbine, and
- fractionation by compression, preferably by means of gas compressor.

The preliminary stage can comprise combining at least two successive fractionations of an identical or different type, in particular chosen from the six above-mentioned types of fractionation.

Any type of fractionation that is useful in general for separating ethane alone or optionally a mixture with the ($C_{3+}$) alkanes from the natural gas, can be utilized in the preliminary stage.

In the process of the present invention, stage (1) comprises simultaneous ethane self- and cross-metathesis reactions carried out by contacting ethane optionally mixed with one or more other alkanes (such as the above-mentioned alkanes) with a metal catalyst (C1) capable of producing, in contact with alkane, reactions involving the splitting and recombining of carbon-carbon and/or carbon-hydrogen and/or carbon-metal bonds, so as to form a reaction mixture (M1) comprising methane and a mixture of the ($C_{4+}$), preferably the ($C_{5+}$) alkanes, and optionally unreacted ethane, propane and where applicable butane(s) (in particular when a liquid mixture of the ($C_{5+}$) is preferably prepared according to the present process).

The catalyst (C1) can be chosen from metal catalysts which are capable of alkane metathesis, and preferably selected from alkane metathesis catalysts. It can be chosen in particular from supported metal clusters and preferably from metal hydrides, organometallic compounds and organometallic hydrides, particularly supported on, and more specifically grafted onto, a solid support.

The catalyst (C1) can be chosen from supported metal clusters comprising one or more metals of Groups 4, 5 and/or 6 of the Periodic Table of the Elements, preferably tantalum. The Table (here and hereafter) is that proposed by IUPAC in 1991 and, for example, published by CRC Press, Inc. (USA) in "CRC Handbook of Chemistry and Physics", 76th Edition, David R. Lide (1995-1996). The supported metal clusters generally comprise a solid support and a metal cluster, and preferably comprise bonding between the metals of the metal cluster, and also bonding between the metal cluster and the solid support. Thus, the metal atom of the metal cluster can be bonded to at least one other metal atom of the metal cluster, preferably to six or fewer metal atoms of the metal cluster. The metal atom of the metal cluster can be bonded in addition to at least one hydrocarbon radical and/or to at least one hydrogen atom. The solid support can be chosen from a metal oxide, a refractory oxide, a molecular sieve, a zeolite, a metal phosphate and a material comprising a metal and oxygen, preferably silica. More particularly, the supported metal cluster can comprise tantalum (as a metal cluster) and silica (as a solid support), preferably bonding between tantalum and tantalum, and also bonding between tantalum and silica, in particular bonding between tantalum and oxygen on the silica surface. The catalyst (C1) can be chosen from the supported metal clusters described in International Patent Application WO 2006/060692, and prepared according to any of the methods described in said Application.

The catalyst (C1) is preferably chosen from metal hydrides, organometallic compounds and organometallic hydrides, particularly supported on, and more specifically grafted onto, a solid support. It can comprise at least one metal chosen from lanthanides, actinides and metals of Groups 2 to 12, preferably transition metals of Groups 3 to 12, more particularly Groups 3 to 10 of the Periodic Table of the Elements. In particular, the metal of the catalyst can be chosen from scandium, yttrium, lanthanum, titanium, zirconium, hafnium, vanadium, niobium, tantalum, chromium, molybdenum, tungsten, rhenium, ruthenium, osmium, nickel, iridium, palladium, platinum, cerium and neodymium. It can be chosen, preferably, from yttrium, titanium, zirconium, hafnium, vanadium, niobium, tantalum, chromium, molybdenum, tungsten, rhenium, ruthenium and platinum, and more especially from niobium, tantalum, molybdenum, tungsten and rhenium.

The catalyst (C1) can be chosen from metal hydrides, and preferably from organometallic compounds and organometallic hydrides, comprising at least one hydrocarbon radical (R), either saturated or unsaturated, linear or branched, having preferably from 1 to 20, in particular from 1 to 14 carbon atoms. The hydrocarbon radical (R) can be chosen from alkyl radicals, in particular either saturated or unsaturated, linear or branched, aliphatic or alicyclic, for example alkyl, alkylidene or alkylidyne radicals, particularly from $C_1$ to $C_{10}$, from aryl radicals, in particular from $C_6$ to $C_{12}$, and from aralkyl, aralkylidene or aralkylidyne radicals, particularly from $C_7$ to $C_{14}$. Thus, the metal atom of the catalyst can preferably be bonded to at least one hydrogen atom and/or to at least one hydrocarbon radical (R), in particular by a single, double or triple carbon-metal bond.

The catalyst (C1) preferably selected from metal hydrides, organometallic compounds and organometallic hydrides, can also comprise one or more ligands, such as "ancillary" ligands, preferably comprising at least one oxygen atom and/or at least one nitrogen atom, and chosen more particularly from oxo, alkoxo, aryloxo, aralkyloxo, nitrido, imido and amido ligands. Generally, by oxo, alkoxo, aryloxo, aralkyloxo, nitrido, imido and amido ligands are respectively meant:

- a divalent oxo radical of general formula : = O
- a monovalent alkoxo, aryloxo or aralkyloxo radical of general formula : - OR'
- a trivalent nitrido radical of general formula : ≡ N
- a divalent imido radical of general formula : = NR", and
- a monovalent amido radical of general formula:- $NR^1R^2$,

general formulae in which O represents an oxygen atom, N represents a nitrogen atom, R' represents an hydrogen atom or a monovalent hydrocarbon radical selected respectively from alkyl, aryl and aralkyl radicals, R" represents an hydrogen atom or a monovalent hydrocarbon radical, and $R^1$ and $R^2$, being identical or different, represent an hydrogen atom or a monovalent hydrocarbon radical. More particularly, R', R", $R^1$ and $R^2$ can be a monovalent hydrocarbon radical, either saturated or unsaturated, linear or branched, comprising from 1 to 20, preferably from 1 to 14 carbon atoms, and particularly chosen from alkyl radicals, preferably from $C_1$ to $C_{10}$, aralkyl radicals, preferably from $C_7$ to $C_{14}$, and aryl radicals, preferably from $C_6$ to $C_{12}$.

Thus, in the catalyst (C1) which is preferably selected from metal hydrides, organometallic compounds and organometallic hydrides, and which can comprise at least one of the above-mentioned ligands, the metal of the catalyst can be bonded to at least one hydrogen atom and/or to at least one hydrocarbon radical (R), and in addition to at least one of these ligands, e.g. to the O atom of the oxo radical by a double bond, or to the O atom of the alkoxo, aryloxo or aralkyloxo radical (OR') by a single bond, or to the N atom of the nitrido radical by a triple bond, or to the N atom of the imido radical (NR") by a double bond, or to the N atom of the amido radical ($NR^1R^2$) by a single bond. Generally, the presence of the oxo, alkoxo, aryloxo, aralkyloxo, nitrido, imido and/or amido ligands in the catalyst (C1) can favourably influence the behaviour of the catalyst in the ethane self- and cross-metathesis reactions of stage (1).

In addition, the catalyst (C1) chosen from metal hydrides, organometallic compounds and organometallic hydrides is preferably supported on, or more particularly grafted onto, a solid support that can be chosen from metal or refractory oxides, sulfides, carbides, nitrides and salts, and from carbon, mesoporous materials, organic/inorganic hybrid materials, metals and molecular sieves. The solid support is in particular chosen from silicas, aluminas, silica-aluminas and aluminium silicates, either simple or modified by other metals, and from zeolites, clays, titanium oxide, cerium oxide, magnesium oxide, niobium oxide, tantalum oxide and zirconium oxide. It can be chosen, preferably, from silicas, aluminas, silica-aluminas and aluminium silicates, either simple or modified by other metals. The solid support can have a specific surface area (B.E.T.) measured according to the standard ISO 9277 (1995) which is chosen in a range of from 0.1 to 5000 $m^2/g$, preferably from 1 to 3000 $m^2/g$, particularly from 1 to 1000 $m^2/g$. It is preferred to use a catalyst (C1) grafted onto a solid support and in which the metal atom can be bonded to at least one atom of hydrogen and/or to at least one hydrocarbon radical (R), and also to at least one of the atoms of the support, in particular to at least one atom of oxygen, sulfur, carbon or nitrogen of the support, in particular when the support is chosen from metal or refractory oxides, sulfides, carbides, nitrides and salts. The catalyst (C1) can be preferably a metal hydride or an organometallic hydride grafted onto a solid support, more particularly chosen from niobium hydrides or organometallic niobium hydrides grafted onto silica, tantalum hydrides or organometallic tantalum hydrides grafted onto silica, molybdenum hydrides or organometallic molybdenum hydrides grafted onto silica, tungsten hydrides or organometallic tungsten grafted onto alumina, and rhenium hydrides or organometallic rhenium grafted onto silica.

The catalyst (C1) chosen from metal hydrides, organometallic compounds and organometallic hydrides can be in particular selected from the metal catalysts described in International Patent Applications WO 98/02244, WO 03/061823 and WO 2004/089541, and prepared according to any of the methods described in said Applications.

Stage (1) preferably comprises contacting the catalyst (C1) with ethane optionally in a mixture with one or more other alkanes preferably selected from ($C_{3+}$) alkanes (having at least 3 carbon atoms), in particular the ($C_{3+}$) alkanes e.g. the $C_3$ to $C_8$, preferably the $C_3$ to $C_7$, especially the $C_3$ to $C_6$ alkanes of natural gas (from which ethane can be issued), preferably in the above-mentioned molar proportions. The contacting can be performed in various ways in a reaction zone (Z1), either discontinuously (or intermittently) or preferably continuously. More particularly, the contacting can be performed by adding the ethane (optionally in said mixture with one or more other alkanes) to the catalyst (C1), or conversely by adding the catalyst (C1) to the ethane (optionally in said mixture with one or more other alkanes), or else simultaneously by adding the catalyst (C1) and the ethane (optionally in said mixture with one or more other alkanes) to the reaction zone (Z1).

Stage (1) is preferably performed so that ethane reacts with itself in contact with the catalyst (C1), and generally produces methane and propane by an ethane self-metathesis reaction, in particular according to the following equation (2):

$$2\,C_2H_6 \; \rightarrow \; CH_4 \; + \; C_3H_8 \qquad (2)$$

Stage (1) is also preferably performed so that simultaneously ethane reacts with the propane previously obtained and optionally present in the mixture used in stage (1) of ethane with one or more other alkanes, preferably the ($C_{3+}$) alkanes of the natural gas from which ethane can be issued. Generally, it results in a production of methane and butane (s) by an ethane cross-metathesis reaction, in particular according to the following equation (3):

$$C_2H_6 \; + \; C_3H_8 \; \rightarrow \; CH_4 \; + \; C_4H_{10} \qquad (3)$$

Stage (1) is furthermore performed so that ethane preferably continues reacting with successively higher alkanes having at least 4 carbon atoms, previously obtained by preceding ethane cross-metathesis reactions and optionally present in the mixture used in stage (1) of ethane with one or more other alkanes, preferably the ($C_{3+}$) alkanes of the natural gas from which ethane can be issued. Generally, it results in the production of methane and successively higher alkanes having one additional carbon atom compared with the previously involved higher alkanes, by successive ethane cross-metathesis reactions, for example, between ethane and respectively butane(s), pentane(s), hexane(s) and heptane (s), in particular according to the following equations (4) to (7)

$$C_2H_6 \; + \; C_4H_{10} \; \rightarrow \; CH_4 \; + \; C_5H_{12} \qquad (4)$$

$$C_2H_6 + C_5H_{12} \rightarrow CH_4 + C_6H_{14} \quad (5)$$

$$C_2H_6 + C_6H_{14} \rightarrow CH_4 + C_7H_{16} \quad (6)$$

$$C_2H_6 + C_7H_{16} \rightarrow CH_4 + C_8H_{18} \quad (7)$$

It is thus found that in stage (1), simultaneous ethane self- and cross-metathesis reactions are performed so as to convert ethane into the desired mixture of the ($C_{4+}$), preferably the ($C_{5+}$) alkanes. It is remarkable to note that in stage (1), ethane can be preferably used in a mixture with one or more other alkanes, preferably the ($C_{3+}$) alkanes, e.g. the $C_3$ to $C_8$, preferably the $C_3$ to $C_7$, especially the $C_3$ to $C_6$ alkanes of the natural gas from which ethane can be issued, since these alkanes used in stage (1) in a mixture with ethane are capable of promoting the development of the simultaneous ethane cross-metathesis reactions, in particular towards an optimum production of the desired mixture of the ($C_{4+}$), preferably the ($C_{5+}$) alkanes. It is also remarkable to note that the present process can be carried out with a high overall yield, in particular at a very high carbon atom efficiency, preferably when methane produced in stage (1) is then separated and used in stages (2) and (3) of the process. It should also be noted that in addition, ($C_{4+}$) or preferably ($C_{5+}$) alkane cross-metathesis reactions can be also simultaneously involved in stage (1) with other ($C_{4+}$) or preferably ($C_{5+}$) alkanes, so as to continue producing higher alkanes.

Stage (1) thus leads to the formation of a reaction mixture (M1) comprising methane and the mixture of the ($C_{4+}$), preferably the ($C_{5+}$) alkanes, and optionally unreacted ethane and propane, and where applicable butane(s). Under the operating conditions of stage (1), the reaction mixture (M1) is generally obtained in a gaseous form or in a mixed gaseous/liquid form.

Stage (1) can be advantageously performed in conditions suited to promoting the development of the simultaneous ethane self- and cross-metathesis reactions, such as those described above, in particular towards an optimum production of the desired mixture of the ($C_{4+}$), preferably the ($C_{5+}$) alkanes. Stage (1) can be performed at a temperature chosen in a range of from 20 to 400°C, preferably from 50 to 350°C, particularly from 70 to 300°C, in particular from 100 to 250°C. It can also be performed under an absolute total pressure chosen in a range of from 0.1 to 50 MPa, preferably from 0.1 to 30 MPa, particularly from 0.1 to 20 MPa. It can also be performed in the presence of at least one inert agent, either liquid or gaseous, in particular of at least one inert gas preferably chosen from nitrogen, helium and argon. Preferably, stage (1) is performed in the presence of hydrogen, in particular under a partial pressure of hydrogen chosen in a range of from 0.0001 to 10 MPa, preferably from 0.001 to 1 MPa, since it is observed that the presence of hydrogen in stage (1) generally increases the activity of the catalyst (C1) and the production of the desired mixture of the ($C_{4+}$), preferably the ($C_{5+}$) alkanes.

Stage (1) can be performed in various ways, either intermittently (or discontinuously), or preferably continuously, more particularly in a reaction zone (Z1) which can be either distinct and separate from fractionation zone (F1) wherein stage (2) is performed, e.g. in at least one reactor, or arranged in a part of said fractionation zone (F1), e.g. in a distillation/condensation column reactor, such as described in American Patent US 4,242,530.

The reaction zone (Z1) can comprise at least one reactor chosen from static reactors, recycling reactors and dynamic reactors. For example, stage (1) can be performed in a static reactor containing fixed quantities of ethane (or of the mixture of ethane with one or more other alkanes) and of catalyst (C1). Stage (1) can also be performed in a recycling reactor into which ethane (or the mixture of ethane with one or more other alkanes) or at least one component of the reaction mixture (M1), e.g. methane, the mixture of the ($C_{4+}$), preferably the ($C_{5+}$) alkanes and optionally unreacted ethane and propane, and where applicable butanes, can be recycled preferably continuously. Stage (1) can also be performed in a dynamic reactor containing the catalyst (C1), preferably in the form of solid particles, through which ethane (or the mixture of ethane with one or more other alkanes) can pass or circulate, preferably continuously.

In practice, stage (1) can be performed in a reaction zone (Z1) comprising at least one reactor chosen from tubular (or multi-tubular) reactors, distillation/condensation column reactors, slurry reactors, fluidised bed reactors, mechanically agitated bed reactors, fluidised and mechanically agitated bed reactors, fixed bed reactors, moving bed reactors and circulating bed reactors. The catalyst (C1) is generally solid, and can optionally be mixed with an inert solid agent chosen in particular from silicas, aluminas, silica-aluminas and aluminium silicates, either simple or modified by other metals. It can be arranged inside the tube(s) of a tubular (or multi-tubular) reactor. It can also be arranged inside a distillation/condensation column reactor, wherein the catalyst is preferably a component of a distillation system functioning as both a catalyst and a distillation packing, i.e. a packing for a distillation column having both a distillation function as in stage (2), and a catalytic function as in stage (1): for example, rings, saddles, balls, irregular shapes, granulates, sheets, tubes, spirals, packed in bags, as described in American Patent US 4,242,530. The catalyst (C1) can also form the bed of a fluidised and/or mechanically agitated bed, a fixed bed, a moving bed or a circulating bed of said reactors. Ethane (or the mixture of ethane with one or more other alkanes) can be introduced into one of said reactors, preferably continuously, and generally can pass or circulate preferably continuously in the form of a gaseous stream into the tube(s), or through the bed or the distillation packing of said reactors. In order to promote the development of the simultaneous ethane self- and cross-metathesis reactions towards an optimum production of the desired mixture of the ($C_{4+}$), preferably the ($C_{5+}$) alkanes, stage (1) can advantageously be performed by withdrawing said mixture of the ($C_{4+}$), preferably the ($C_{5+}$) alkanes and/or the methane out of the reaction zone (Z1), preferably continuously.

Thus, for example, stage (1) can be performed:

(i) by continuously introducing ethane (or ethane in a mixture with one or more other alkanes) into stage (1), in particular into the reaction zone (Z1) containing the catalyst (C1), so as to form continuously the reaction mixture (M1) generally comprising methane and the mixture of the ($C_{4+}$), preferably the ($C_{5+}$) alkanes, and optionally unreacted ethane and propane, and where applicable butane(s), and

(ii) by continuously withdrawing at least a part of the reaction mixture (M1) out of stage (1), in particular out of the reaction zone (Z1), so as to subject said part of the reaction mixture (M1) to stage (2) and to stage (6) preferably for separating the mixture of the ($C_{4+}$), preferably the ($C_{5+}$) alkanes from the reaction mixture (M1) and isolating the liquid mixture of the ($C_{4+}$), preferably ($C_{5+}$) alkanes from the rest of the reaction mixture which is preferably returned to stage (1), in particular into the reaction zone (Z1), more particularly in order to go on with the simultaneous ethane self- and cross-metathesis reactions and continuing forming said reaction mixture (M1).

The process comprises stage (2) comprising separating methane from the reaction mixture (M1), so as to isolate the methane. Stage (2) is preferably performed by fractionating the reaction mixture (M1) into (a) methane and (b) the rest of the reaction mixture which generally comprises the mixture of the ($C_{4+}$), preferably the ($C_{5+}$) alkanes, and optionally the unreacted ethane, propane and where applicable butane(s), so as to isolate (a) the methane and (b) the rest of the reaction mixture which is preferably returned to stage (1), in particular into the reaction zone (Z1). Stage (2) can be performed in various ways, either intermittently (or discontinuously) or preferably continuously. It can comprise one or more methods of fractionation which are generally useful for separating methane from natural gas, e.g. any method or combination of methods for separating methane from the reaction mixture (M1) comprising methane and the mixture of the ($C_{4+}$), preferably the ($C_{5+}$) alkanes, and optionally (unreacted) ethane, propane and where applicable butane(s). The methods of fractionation can be chosen from the following six types of fractionation:

- fractionation by change of physical state, preferably of gaseous/liquid phase, in particular by distillation or by condensation (or liquefaction), more particularly by partial condensation (or partial liquefaction), preferably by means of distillation/condensation column or column-reactor,
- fractionation by molecular filtration, preferably by means of semi-permeable and selective membrane,
- fractionation by adsorption, preferably by means of molecular sieve or any other adsorbent,
- fractionation by absorption, preferably by means of absorbing oil,
- fractionation by cryogenic expansion, preferably by means of expansion turbine, and
- fractionation by compression, preferably by means of gas compressor.

Stage (2) thus makes it possible to separate and to isolate (a) methane and (b) the rest of the reaction mixture (M1) which is preferably returned to stage (1), in particular into the reaction zone (Z1). It can be performed in a fractionation zone (F1) which generally is either separate and distinct from the reaction zone (Z1) wherein stage (1) is performed, more particularly comprising at least one distillation/condensation column or tower, or arranged in a part of said reaction zone (Z1), e.g. in a distillation/condensation column reactor, as described in American patent US 4,242,530.

More generally, the fractionation in stage (2) can be essentially performed by change of the physical state, preferably of the gaseous/liquid phase of the reaction mixture (M1), particularly by distillation or by condensation (or liquefaction), more particularly by partial condensation (or by partial liquefaction), e.g. in at least one distillation/condensation column or tower, or in a distillation/condensation column reactor. The fractionation in stage (2) can be performed by refrigeration or cryogenic processes, preferably by cooling of the reaction mixture (M1) to a temperature at which a fractionated mixture comprising the mixture of the ($C_{4+}$), preferably the ($C_{5+}$) alkanes, and optionally the unreacted ethane, propane and where applicable butane(s) condense, so as to condense said fractionated mixture and to separate and to isolate the methane in gaseous form.

Generally, the fractionation in stage (2) can be also performed by molecular filtration, in particular by passing the reaction mixture (M1) through at least one semi-permeable and selective membrane, so as to arrest and to isolate the mixture of the ($C_{4+}$), preferably the ($C_{5+}$) alkanes, and optionally the unreacted ethane, propane and where applicable butane(s), and to allow the methane to pass through and to be isolated in its turn in gaseous form.

The semi-permeable and selective membrane can be chosen from membranes suitable for separating methane from natural gas, e.g. membranes preferably chosen from zeolite membranes, porous alumina membranes, ceramic membranes, flowing liquid membranes and supported liquid membranes.

Generally, the fractionation in stage (2) can be also performed by adsorption, in particular by passing the reaction mixture (M1) onto at least one molecular sieve or any other adsorbent, so as to retain the methane (or the rest of the reaction mixture (M1)), and to allow said rest of the reaction mixture (M1) (or the methane) to pass through, and then by subjecting the methane (or said rest of the reaction mixture (M1)) thus retained to at least one desorption step, preferably by the TSA ("Temperature Swing Adsorption") method or by the PSA ("Pressure Swing Adsorption") method, so as to isolate said methane (or said rest of the reaction mixture (M1)).

The adsorbents can be chosen from adsorbents suitable for retaining either methane, or the rest of the reaction mixture (M1), e.g. adsorbents preferably chosen from molecular sieves, silica gels, activated charcoals and activated carbons.

Generally, the fractionation in stage (2) can be also performed by absorption, in particular by contacting the reaction mixture (M1) with an absorption oil, e.g. in an absorption tower, so as to soak up the fractionated mixture comprising the mixture of the $(C_{4+})$, preferably the $(C_{5+})$ alkanes and optionally the unreacted ethane, propane and where applicable butane(s), and to form an oil/alkane mixture, and then by subjecting said oil/alkane mixture, e.g. in a recovery tower, to a temperature above the boiling point of said fractionated mixture, but below that of the absorbing oil, so as to separate and to isolate said fractionated mixture from the absorbing oil and from the methane which is thus separated and isolated in its turn.

Generally, the fractionation in stage (2) can be also performed by a cryogenic expansion, in particular by dropping the temperature of the reaction mixture (M1) to a temperature preferably in a range of from -80 to -90°C, in particular around -85°C, more particularly by use of external refrigerants to cool the reaction mixture (M1) and then by means of an expansion turbine which rapidly expands the chilled reaction mixture (M1) and causes the temperature to drop significantly, so as to condense the fractionated mixture comprising the mixture of the $(C_{4+})$, preferably the $(C_{5+})$ alkanes and optionally the unreacted ethane, propane and where applicable butane(s), to separate and to isolate said fractionated mixture in liquid form from the methane which is isolated in its turns in gaseous form.

Generally, the fractionation in stage (2) can be also performed by compression, in particular by compressing the reaction mixture (M1), e.g. by means of a gas compressor, so as to condense the fractionated mixture comprising the mixture of the $(C_{4+})$, preferably the $(C_{5+})$ alkanes and optionally the unreacted ethane, propane and where applicable butane(s), to separate and to isolate said fractionated mixture in liquid form from the methane which is isolated in its turn in gaseous form.

Stage (2) can also comprise combining two or more successive fractionations of an identical or different type, in particular fractionations chosen from the six above-mentioned types of fractionation, in a chronological order or in a reverse order.

Stage (3) of the process comprises a non-oxidative methane coupling reaction carried out by contacting the methane isolated in stage (2) with a metal catalyst (C2) capable of producing, in contact with alkane, reactions involving the splitting and recombining of carbon-carbon and/or carbon-hydrogen and/or carbon-metal bonds, said catalyst being identical to or different from the metal catalyst (C1) in stage (1), so as to form a mixture (M2) comprising ethane and hydrogen, and optionally unreacted methane.

The catalyst (C2) can be identical to or preferably different from the catalyst (C1) used in stage (2), and can conform in particular to the same definitions of metal catalysts given above for the catalyst (C1).

In particular, the catalyst (C2) can be chosen from metal catalysts that are capable of alkane metathesis, and preferably selected from alkane metathesis catalysts. It can be chosen in particular from supported metal clusters, and preferably from metal hydrides, organometallic compounds and organometallic hydrides, particularly supported on, more specifically grafted onto, a solid support.

The catalyst (C2) can be chosen from supported metal clusters comprising one or more metals of Groups 4, 5 and/or 6 of the Periodic Table of the Elements, preferably tantalum. The supported metal clusters generally comprise a solid support and a metal cluster, and preferably comprise bonding between the metals of the metal cluster, and also bonding between the metal cluster and the solid support. Thus, the metal atom of the metal cluster can be bonded to at least one other metal atom of the metal cluster, preferably to six or fewer metal atoms of the metal cluster. The metal atom of the metal cluster can be bonded in addition to at least one hydrocarbon radical and/or to at least one hydrogen atom. The solid support can be chosen from a metal oxide, a molecular sieve, a zeolite, a metal phosphate and a material comprising a metal and oxygen, and preferably silica. More particularly, the supported metal cluster can comprise tantalum (as a metal cluster) and silica (as a solid support), preferably bonding between tantalum and tantalum, and also bonding between tantalum and silica, in particular bonding between tantalum and oxygen on the silica surface. The catalyst (C2) can be chosen from the supported metal clusters described in International Patent Application WO 2006/060692, and prepared according to any methods described in said Application.

The catalyst (C2) is preferably chosen from metal hydrides, organometallic compounds and organometallic hydrides, particularly supported on, and more especially grafted onto a solid support. It can comprise at least one metal preferably chosen from scandium, yttrium, lanthanum, titanium, zirconium, hafnium, vanadium, niobium, tantalum, chromium, molybdenum, tungsten, rhenium, ruthenium, osmium, nickel, iridium, palladium, platinum, cerium and neodymium. The metal of the catalyst (C2) can be preferably chosen from yttrium, titanium, zirconium, hafnium, vanadium, niobium, tantalum, chromium, molybdenum, tungsten, rhenium, ruthenium and platinum, and more especially chosen from niobium, tantalum, molybdenum, tungsten and rhenium.

The catalyst (C2) can be chosen from metal hydrides, and preferably from organometallic compounds and organometallic hydrides, comprising more particularly at least one hydrocarbon radical (R), either saturated or unsaturated, linear or branched, preferably comprising from 1 to 20, particularly from 1 to 14 carbon atoms. The hydrocarbon radical (R) can be chosen from alkyl radicals, preferably either saturated or unsaturated, linear or branched, aliphatic or alicyclic, for example alkyl, alkylidene or alkylidyne radicals, more particularly from $C_1$ to $C_{10}$, from aryl radicals, more particularly from $C_6$ to $C_{12}$, and from aralkyl, aralkylidine or aralkylidyne radicals, more particularly from $C_7$ to $C_{14}$. Thus, the metal atom of the catalyst (C2) is preferably bonded to at least one hydrogen atom and/or to at least one hydrocarbon radical (R), in particular by a single, double or triple carbon-metal bond.

The catalyst (C2) preferably selected from metal hydrides, organometallic compounds and organometallic hydrides, can also comprise one or more ligands, such as "ancillary" ligands, preferably comprising at least one oxygen atom and/or at least one nitrogen atom, and particularly chosen from oxo, alkoxo, aryloxo, aralkyloxo, nitrido, imido and amido ligands. Generally, by oxo, alkoxo, aryloxo, aralkyloxo, nitrido, imido and amido ligands are respectively meant:

- a divalent oxo radical of general formula : $= O$
- a monovalent alkoxo, aryloxo or aralkyloxo radical of general formula: $- OR'$
- a trivalent nitrido radical of general formula : $\equiv N$
- a divalent imido radical of general formula : $= NR''$, and
- a monovalent amido radical of general formula: $- NR^1R^2$,

general formulae in which O represents an oxygen atom, N represents a nitrogen atom, R' represents an hydrogen atom or a monovalent hydrocarbon radical selected respectively from alkyl, aryl and aralkyl radicals, R'' represents an hydrogen atom or a monovalent hydrocarbon radical, and $R^1$ and $R^2$, being identical or different, represent a monovalent hydrocarbon radical. More particularly, R', R'', $R^1$ and $R^2$ can be a monovalent hydrocarbon radical, either saturated or unsaturated, linear or branched, in particular comprising from 1 to 20, preferably from 1 to 14 carbon atoms, and more particularly chosen from alkyl radicals, preferably from $C_1$ to $C_{10}$, aryl radicals from $C_6$ to $C_{12}$ and aralkyl radicals, from $C_7$ to $C_{14}$.

Thus, in the catalyst (C2) which is preferably selected from metal hydrides, organometallic compounds and organometallic hydrides, and which can comprise at least one of the above-mentioned ligands, the metal of the catalyst can be bonded to at least one hydrogen atom and/or to at least one hydrocarbon radical (R), and in addition to at least one of these ligands, e.g. to the O atom of the oxo radical by a double bond, or to the O atom of the alkoxo, aryloxo or aralkyloxo radical (OR') by a single bond, or to the N atom of the nitrido radical by a triple bond, or to the N atom of the imido radical (NR'') by a double bond, or to the N atom of the amido radical ($NR^1R^2$) by a single bond. Generally, the presence of the oxo, alkoxo, aryloxo, aralkyloxo, nitrido, imido and/or amido ligands in the catalyst (C2) can favourably influence the behaviour of the catalyst in the methane coupling reaction of stage (3).

In addition, the catalyst (C2) chosen from metal hydrides, organometallic compounds and organometallic hydrides is preferably supported on, more particularly grafted onto, a solid support that can be chosen from metal or refractory oxides, sulfides, carbides, nitrides and salts, and from carbon, mesoporous materials, organic/inorganic hybrid materials, metals and molecular sieves. The solid support can be chosen from silicas, aluminas, silica-aluminas and aluminium silicates, either simple or modified by other metals, and from zeolites, clays, titanium oxide, cerium oxide, magnesium oxide, niobium oxide, tantalum oxide and zirconium oxide. It can be preferably chosen from silicas, aluminas, silica-aluminas and aluminium silicates, either simple or modified by other metals. The catalyst (C2) is preferably a metal hydride or an organometallic hydride grafted onto a solid support, more especially chosen from niobium hydrides or organometallic niobium hydrides grafted onto silica, tantalum hydrides or organometallic tantalum hydrides grafted onto silica, molybdenum hydrides or organometallic molybdenum hydrides grafted onto silica, tungsten hydrides or organometallic tungsten hydrides grafted onto alumina, and rhenium hydrides or organometallic rhenium hydrides grafted onto silica.

The catalyst (C2) chosen from metal hydrides, organometallic compounds and organometallic hydrides can be in particular chosen from the metal catalysts described in the International Patent Applications WO 98/02244, WO 03/061823 and WO 2004/089541, and prepared according to any of the methods described in said applications.

Stage (3) is preferably performed so that methane reacts with itself in contact with the catalyst (C2), and generally produces ethane and hydrogen by a non-oxidative coupling reaction (i.e. a homologation reaction), in particular described according to the following equation (8):

$$2\,CH_4 \;\rightarrow\; C_2H_6 \;+\; H_2 \qquad (8)$$

Stage (3) generally leads to formation of a mixture (M2) comprising ethane and hydrogen, and optionally unreacted methane. Under the operating conditions of stage (3), the mixture (M2) is generally obtained in gaseous form.

Stage (3) can be performed under conditions specially suited to promoting the development of the methane coupling reaction towards an optimum production of ethane. Thus, it can be performed at a temperature chosen in a range of from 150 to 700°C, preferably from 200 to 650°C, more particularly from 300 to 600°C. It can also be performed under an absolute total pressure chosen in a range of from 0.1 to 50 MPa, preferably from 0.1 to 30 MPa, more particularly from 0.1 to 20 MPa. It can also be performed in the presence of at least one inert agent, either liquid or gaseous, in particular of at least one inert gas preferably chosen from nitrogen, helium and argon.

Stage (3) can be performed in various ways, either intermittently (or discontinuously) or preferably continuously, in a reaction zone (Z2). Stage (3) can comprise adding the methane to the catalyst (C2), or adding the catalyst (C2) to the methane, or else simultaneously adding the methane and the catalyst (C1) to the reaction zone (Z2).

The reaction zone (Z2) can be either separate and distinct from fractionation zones (F1) and (F2) wherein stage (2) and stage (4) are respectively performed, e.g. in at least one reactor, or arranged in a part of said fractionation zone (F1) or preferably of said fractionation zone (F2), e.g. in a distillation/condensation column reactor, such as described in American Patent US 4,242,530.

In addition, the reaction zone (Z2) can comprise at least one reactor chosen from static reactors, recycling reactors and dynamic reactors. For example, stage (3) can be performed in a static reactor containing fixed quantities of methane and of catalyst (C2). Stage (3) can be also performed in a recycling reactor into which methane or at least one component of the mixture (M2), for example ethane or optionally unreacted methane, can be recycled, preferably continuously. Stage (3) can be also performed in a dynamic reactor containing the catalyst (C2), particularly in the form of solid particles, through which methane can pass or circulate preferably continuously.

In practice, stage (3) can be performed in a reaction zone (Z2) comprising at least one reactor chosen from tubular (or multi-tubular) reactors, distillation/condensation column reactors, slurry reactors, fluidised bed reactors, mechanically agitated bed reactors, fluidised and mechanically agitated bed reactors, fixed bed reactors, moving bed reactors and circulating bed reactors. The catalyst (C2) is generally solid, and can optionally be mixed with an inert solid agent chosen in particular from silicas, aluminas, silica-aluminas and aluminium silicates, either simple or modified by other metals. The catalyst (C2) can be arranged inside the tube(s) of a tubular (or multi-tubular) reactor. It can also be arranged inside a distillation/condensation column reactor, wherein the catalyst is preferably a component of a distillation system functioning as both a catalyst and a distillation packing, i.e. a packing for a distillation column having both a distillation function as in stage (2) or preferably in stage (4), and a catalytic function as in stage (3): for example, rings, saddles, balls, irregular shapes, granulates, sheets, tubes, spirals, packed in bags, as described in American Patent US 4,242,530. The catalyst (C2) can also form the bed of a fluidised and/or mechanically agitated bed, a fixed bed, a moving bed or a circulating bed of said reactors. Methane can be introduced in the form of a stream, preferably continuously, into one of said reactors, and generally can pass or circulate into the tube(s), or through the bed or the distillation packing of said reactors. In order to promote the development of the methane coupling reaction towards an optimum production of ethane, stage (3) can be advantageously performed by withdrawing at least one component of the mixture (M2), such as ethane, out of the reaction zone (Z2), preferably continuously.

Thus, for example, stage (3) can be performed:

(i) by continuously introducing methane into stage (3), in particular into the reaction zone (Z2) containing the catalyst (C2), so as to form continuously the mixture (M2) generally comprising ethane and hydrogen, and optionally unreacted methane, and

(ii) by continuously withdrawing at least one part of the mixture (M2) out of stage (3), in particular out of the reaction zone (Z2), so as to submit said part of the mixture (M2) to stage (4) for separating and isolating ethane from the rest of the mixture (M2), and recycling the ethane thus isolated into stage (1), and preferably returning said rest of the mixture, preferably the unreacted methane into stage (3), in particular into the reaction zone (Z2), particularly in order to go on with the methane coupling reaction of stage (3) and to continue forming the mixture (M2).

The process optionally comprises a stage (4) for separating ethane and optionally hydrogen from the mixture (M2), so as to isolate the ethane, optionally the hydrogen and optionally the unreacted methane, or to isolate the ethane in a mixture with the hydrogen. Stage (4) can be performed in various ways, either intermittently (or discontinuously) or preferably continuously. It can comprise one or more methods of fractionation which generally are useful for separating ethane from a mixture comprising ethane, hydrogen and optionally methane. The methods of fractionation can be chosen from any method or combination of methods for fractionation of such a mixture, preferably from the following six types of fractionation:

- fractionation by change of physical state, preferably of gaseous/liquid phase, in particular by distillation or by condensation (or liquefaction), more particularly by partial condensation (or partial liquefaction), preferably by means of distillation/condensation column or column-reactor,
- fractionation by molecular filtration, preferably by means of semi-permeable and selective membrane,
- fractionation by adsorption, preferably by means of molecular sieve or any other adsorbent,
- fractionation by absorption, preferably by means of absorbing oil,
- fractionation by cryogenic expansion, preferably by means of expansion turbine, and
- fractionation by compression, preferably by means of gas compressor.

Stage (4) can furthermore permit the hydrogen to be separated and isolated from the mixture (M2) and preferably to be used for any applications or stages preferably employed in the present process. It can also permit the unreacted methane to be separated and isolated from the mixture (M1) and preferably to be recycled into stage (3).

Stage (4) can be performed in a fractionation zone (F2) which is either separate and distinct from the reaction zone (Z2) wherein stage (3) is performed, preferably comprising at least one distillation/condensation column or tower, or arranged in a part of said reaction zone (Z2), preferably in a distillation/condensation column reactor, such as previously mentioned and described in American Patent US 4,242,530.

Thus, a distillation/condensation column reactor can be advantageously used both for stages (3) and (4), and can contain the catalyst (C2). More particularly, the catalyst (C2) can be arranged inside the distillation/condensation column reactor, as a component of a distillation system functioning as both a catalyst and a distillation packing. Thus, stages (2) and (3) can be simultaneously performed in a distillation/condensation column reactor comprising both a reaction zone and a fractionation zone. In the reaction zone, the contacting of methane with the catalyst (C2) can be performed as in stage (3), so as to form the mixture (M2) preferably in a gaseous form or a mixed gaseous/liquid form. Simultaneously, in the fractionation zone, the separation of ethane from the mixture (M2) can be performed as in stage (4), in particular by distillation or by condensation, more particularly by partial condensation, so as to isolate the ethane.

More generally, a fractionation of the mixture (M2) can be performed by change of the physical state, preferably of the gaseous/liquid phase of the mixture, e.g. in at least one distillation/condensation column or tower, or in a distillation/condensation column reactor, in particular by refrigeration or cryogenic processes. Preferably it can be performed by at least one cooling of the gaseous mixture (M2) to at least one temperature at which at least one component of the mixture condenses and the rest of the mixture remains in gaseous form, so as to condense, to separate and to isolate said component(s) from said rest of the mixture.

Generally, a fractionation of the mixture (M2) can also be performed by molecular filtration of the mixture, preferably by passing the gaseous mixture (M2) through at least one semi-permeable and selective membrane, so as to arrest and to isolate at least one component of the mixture, and to allow the rest of the mixture to pass through.

Generally, a fractionation of the mixture (M2) can also be performed by adsorption, preferably by passing the gaseous mixture (M2) onto at least one molecular sieve or any other adsorbent, so as to retain at least one component of the mixture, and to allow the rest of the mixture to pass through, and by submitting the component(s) thus retained to at least one desorption step, preferably by the TSA or PSA method, so as to isolate said component(s).

Generally, a fractionation of the mixture (M2) can also be performed by absorption, preferably by contacting the gaseous mixture (M2) with an absorption oil, so as to soak up at least one component of the mixture to form an oil/component mixture and to allow the rest of the mixture to be free and not absorbed, then by subjecting said oil/component mixture, e.g. in a recovery tower, to a temperature above the boiling point of the component(s), but below that of the oil, so as to separate and to isolate said component(s), from the oil.

Generally, a fractionation of the mixture (M2) can also be performed by cryogenic expansion, in particular by dropping the temperature of the gaseous mixture (M2) to a temperature so as to condense at least one component of the mixture, while maintaining the rest of the mixture in gaseous form, with the help of an expansion turbine rapidly expanding the gaseous mixture and causing a significant drop of the temperature.

Generally, a fractionation of the mixture (M2) can also be performed by compression, in particular by compressing the gaseous mixture (M2) e.g. by means of a gas compressor, so as to condense at least one component of the mixture, to separate and to isolate said component, and to allow the rest of the mixture to be maintained in gaseous form.

For example, a fractionation of the gaseous mixture (M2) can be performed by change of the gaseous phase of the mixture, e.g. in at least one distillation/condensation column or tower, or in a distillation/condensation column reactor, preferably by a cooling of the gaseous mixture (M2) to a temperature at which ethane condenses, so as to condense the ethane, to separate and to isolate it in liquid form from the rest of the gaseous mixture comprising the hydrogen and optionally the unreacted methane.

For example, a fractionation of the gaseous mixture (M2) can also be performed by molecular filtration, preferably by passing the gaseous mixture (M2) through a semi-permeable and selective membrane, so as to arrest and to isolate the ethane, and to allow the rest of the gaseous mixture comprising the hydrogen and optionally the unreacted methane to pass through.

The semi-permeable and selective membranes can be chosen from any membrane suitable for separating ethane from hydrogen and optionally methane, preferably chosen from zeolite membranes, porous alumina membranes, ceramic membranes, flowing liquid membranes and supported liquid membranes.

For example, a fractionation of the gaseous mixture (M2) can also be performed by adsorption, preferably by passing the gaseous mixture (M2) onto a molecular sieve or any other adsorbent, so as to retain the ethane and to allow the rest of the gaseous mixture comprising the hydrogen and optionally the unreacted methane to pass through, and by submitting the ethane thus retained to a desorption step, preferably by the TSA or the PSA method, so as to isolate the ethane.

The adsorbents can be chosen from any adsorbent suitable for retaining ethane from hydrogen and optionally methane, preferably chosen from molecular sieves, silica gels, activated charcoals and activated carbons.

For example, a fractionation of the gaseous mixture (M2) can also be performed by absorption, preferably by contacting the gaseous mixture (M2) with an absorption oil, e.g. in an absorption tower, so as to soak up the ethane from the mixture and to form an oil/ethane mixture, and then preferably by subjecting said oil/ethane mixture to a temperature above the boiling point of ethane, but below that of the oil, so as to separate and to isolate the ethane from the oil and from the rest of the mixture comprising the hydrogen and optionally the unreacted methane.

For example, a fractionation of the gaseous mixture (M2) can also be performed by cryogenic expansion, preferably by dropping the temperature of the gaseous mixture (M2) in particular by use of external refrigerants to cool the gaseous mixture and then by means of an expansion turbine which rapidly expands the chilled gaseous mixture and causes the temperature to drop significantly, so as to condense the ethane, to separate and to isolate it in liquid form from the rest of the gaseous mixture comprising the hydrogen and optionally the unreacted methane.

For example, a fractionation of the gaseous mixture (M2) can also be performed by compression, preferably by compressing the gaseous mixture (M2), e.g. by means of a gas compressor, so as to condense the ethane, to separate and to isolate it in liquid form from the rest of the gaseous mixture comprising the hydrogen and optionally the unreacted methane.

Stage (4) can also be performed advantageously by a double (or multiple) fractionation of the mixture (M2), comprising a combination of two (or more) successive fractionations of an identical or different type, preferably chosen from the six above-mentioned types of fractionation, so as in particular to separate and to isolate (a) ethane, (b) hydrogen and (c) unreacted methane from the mixture (M2).

The unreacted methane can be advantageously separated and isolated in stage (4) and preferably recycled into stage (3) for going on with the non-oxidative methane coupling. In addition, it may also be particularly advantageous to separate and to isolate the hydrogen which is then preferably used in one or more applications or stages, distinct or not from those of said process. For example, the hydrogen thus isolated can be used as an agent for activation or regeneration of the catalyst(s) (C1) and/or (C2) employed in the process. The activation or the regeneration of the catalyst can be carried out by contacting the catalyst with the hydrogen, either in one of the stages of the process, for example in stage (1) for the simultaneous ethane self- and cross-metathesis reactions, or in a stage distinct and separate from the process. The hydrogen can also be used advantageously in other applications or stages distinct from the present process, for example as a fuel for producing thermal and/or electrical energies, in particular as a fuel in hydrogen fuel cells for producing electrical energy, such energies being preferably employed in the present process, or as a fuel for automobile, or as a reagent in a chemical, petrochemical or refinery plant. It is remarkable to notice that hydrogen produced and isolated in the present process can generally supply all the energy needed for running said process.

For example, it is possible to perform a double fractionation by subjecting the gaseous mixture (M2) comprising ethane, hydrogen and unreacted methane to a combination of two successive fractionations of one and the same type, namely by change of the physical state, preferably of the gaseous/liquid phase of the mixture, e.g. in one or more distillation/condensation columns or towers, or in a distillation/condensation column reactor, for example as follows:

(i) by a first cooling of the gaseous mixture (M2) to a temperature at which ethane condenses, so as to condense the ethane, to separate and to isolate it in liquid form from the rest of the gaseous mixture comprising the hydrogen and the unreacted methane, and
(ii) by a second cooling of said rest of the gaseous mixture, so as to condense the methane, to separate and to isolate it in liquid form from the hydrogen which is preferably isolated in its turn in gaseous form.

For example, it is also possible to perform a double fractionation by subjecting the above-mentioned gaseous mixture (M2) to a combination of two successive fractionations of one and the same type, namely by molecular filtration:

(i) by passing the gaseous mixture (M2) through a first semi-permeable and selective membrane, so as to arrest and to isolate the ethane, and to allow the rest of the gaseous mixture comprising the hydrogen and the unreacted methane to pass through, and

(ii) by passing said rest of the gaseous mixture through a second semi-permeable and selective membrane, so as to arrest and to isolate the methane, and to allow the hydrogen to pass through and preferably to be isolated in its turn in gaseous form.

For example, it is also possible to perform a double fractionation by subjecting the above-mentioned gaseous mixture (M2) to a combination of two successive fractionations of one and the same type, namely by adsorption:

(i) by passing the gaseous mixture (M2) onto a first molecular sieve or any other adsorbent, so as to retain the ethane, and to allow the rest of the gaseous mixture comprising the hydrogen and the unreacted methane to pass through, then

(ii) by passing said rest of the gaseous mixture onto a second molecular sieve or any other adsorbent, so as to retain the methane (or the hydrogen), and to allow the hydrogen (or the methane) to pass through and preferably to be isolated, and

(iii) by respectively submitting the ethane and the methane (or the hydrogen) thus retained to desorption steps, preferably by the TSA or PSA method, so as respectively to isolate the ethane and the methane (or the hydrogen).

For example, it is also possible to perform a double fractionation by subjecting the above-mentioned gaseous mixture (M2) to a combination of two successive fractionations of one and the same type, namely by absorption, e.g. in at least one or two absorption towers:

(i) by contacting the gaseous mixture (M2) with a first absorbing oil, so as to soak up the ethane from the mixture, to form an oil/ethane mixture, and to allow the rest of the gaseous mixture comprising the unreacted methane and the hydrogen to be free in gaseous form,

(ii) by contacting said rest of the gaseous mixture with a second absorbing oil, so as to soak up the methane, to form an oil/methane mixture, and to allow the hydrogen to be free in gaseous form and preferably to be isolated, and

(iii) by subjecting said oil/ethane mixture, e.g. in a recovery tower, to a temperature above the boiling point of ethane, but below that of the oil, so as to separate and to isolate the ethane from the oil, and preferably by subjecting said oil/methane mixture, e.g. in a recovery tower, to a temperature above the boiling point of methane, but below that of the oil, so as to separate and to isolate the unreacted methane from the oil.

For example, it is also possible to perform a double fractionation by subjecting the above-mentioned gaseous mixture (M2) to a combination of two successive fractionations of one and the same type, namely by cryogenic expansion:

(i) by dropping the temperature of the gaseous mixture (M2) by means of an expansion turbine, so as to condense the ethane, to separate and to isolate it in liquid form from the rest of the gaseous mixture comprising the hydrogen and the unreacted methane, and

(ii) by dropping the temperature of said rest of the gaseous mixture by means of an expansion turbine, so as to condense the methane, to separate and to isolate it in liquid form from the hydrogen which is preferably isolated in its turn in gaseous form.

For example, it is also possible to perform a double fractionation by subjecting the above-mentioned gaseous mixture (M2) to a combination of two successive fractionations of one and the same type, namely by compression:

(i) by compressing the gaseous mixture (M2), e.g. by means of a gas compressor, so as to condense the ethane, to separate and to isolate it in liquid form from the rest of the gaseous mixture comprising the unreacted methane and the hydrogen, and

(ii) by compressing said rest of the gaseous mixture, e.g. by means of a gas compressor, so as to condense the methane, to separate and to isolate it in liquid form from the hydrogen which is preferably isolated in its turn in gaseous form.

It is also possible to perform a double (or multiple) fractionation by subjecting the mixture (M2), comprising ethane, hydrogen and unreacted methane, to a combination of two (or more) successive fractionations of a different type, preferably chosen from the six above-mentioned types of fractionation.

More particularly, a double fractionation of the above-mentioned mixture (M2) can be performed by combining two successive fractionations of a different type, namely:

- the one by change of the physical state, preferably of the gaseous/liquid phase of the mixture, in particular by distillation or by condensation, and the other by molecular filtration, preferably by means of a semi-permeable and selective membrane, or
- the one by change of the physical state, preferably of the gaseous/liquid phase of the mixture, in particular by distillation or by condensation, and the other by adsorption, preferably by means of a molecular sieve or any other adsorbent, or
- the one by change of the physical state, preferably of the gaseous/liquid phase of the mixture, in particular by distillation or by condensation, and the other by absorption, preferably by means of an absorbing oil, or
- the one by change of the physical state, preferably of the gaseous/liquid phase of the mixture, in particular by distillation or by condensation, and the other by cryogenic expansion, preferably by means of an expansion turbine, or
- the one by change of the physical state, preferably of the gaseous/liquid phase of the mixture, in particular by distillation or by condensation, and the other by compression, preferably by means of gas compressor, or
- the one by molecular filtration, preferably by means of a semi-permeable and selective membrane, and the other by adsorption, preferably by means of a molecular sieve or any other adsorbent, or
- the one by molecular filtration, preferably by means of a semi-permeable and selective membrane, and the other by absorption, preferably by means of an absorbing oil, or
- the one by molecular filtration, preferably by means of a semi-permeable and selective membrane, and the other by cryogenic expansion, preferably by means of an expansion turbine, or
- the one by molecular filtration, preferably by means of a semi-permeable and selective membrane, and the other by compression, preferably by means of gas compressor, or
- the one by adsorption, preferably by means of a molecular sieve or any other adsorbent, and the other by absorption, preferably by means of an absorbing oil, or
- the one by adsorption, preferably by means of a molecular sieve or any other adsorbent, and the other by cryogenic expansion, preferably by means of an expansion turbine, or
- the one by adsorption, preferably by means of a molecular sieve or any other adsorbent, and the other by compression, preferably by means of gas compressor, or
- the one by absorption, preferably by means of an absorbing oil, and the other by cryogenic expansion, preferably by means of an expansion turbine, or
- the one by absorption, preferably by means of an absorbing oil, and the other by compression, preferably by means of gas compressor, or
- the one by cryogenic expansion, preferably by means of an expansion turbine, and the other by compression, preferably by means of gas compressor.

Each of said combinations can be performed in a chronological order or in a reverse order.

Thus, for example, the two successive fractionations of a different type can be performed advantageously:

(i) by cooling the gaseous mixture (M2) to a temperature at which ethane condenses, so as to condense the ethane, to separate and to isolate it in liquid form from the rest of the gaseous mixture comprising the hydrogen and the unreacted methane, and

(ii) by passing said rest of the gaseous mixture through a semi-permeable and selective membrane, so as to arrest and to isolate the methane and to allow the hydrogen to pass through and preferably to be isolated in its turn in gaseous form.

For example, it is also possible to perform said two successive fractionations of a different type, but in a reverse order, that is to say:

(i) by passing the gaseous mixture (M2) through a semi-permeable and selective membrane, so as to arrest and to isolate the ethane and to allow the rest of the gaseous mixture comprising the hydrogen and the unreacted methane to pass through, and

(ii) by cooling said rest of the gaseous mixture to a temperature at which methane condenses, so as to condense the unreacted methane, and to separate and to isolate it in liquid form from the hydrogen which is preferably isolated in its turn in gaseous form.

Thus, by means of a double (or multiple) fractionation such as one of those described above, the unreacted methane thus isolated in stage (4) is preferably recycled into stage (3) for methane coupling. Furthermore, the hydrogen thus isolated in stage (4) is preferably used in at least one of the above-mentioned applications or stages, e.g. as an agent for activation or regeneration of the catalyst(s) (C1) and/or (C2), or as a fuel for producing thermal and/or electrical energies, in particular as a fuel in hydrogen fuel cells for producing electrical energy, such energies being preferably supplied for running the present process, or as a fuel for automobile, or as a reagent in a chemical, petrochemical or refinery plant.

The process also comprises a stage (5) comprising recycling the ethane and optionally the hydrogen isolated in stage (4) or the mixture (M2) obtained in stage (3) into stage (1) especially for going on with the simultaneous ethane self- or cross-metathesis reactions and for continuing forming the reaction mixture (M1). Stage (5) comprises recycling into stage (1) the ethane isolated in stage (4) preferably for promoting the development of the simultaneous ethane self- and cross-metathesis reactions and the production of the reaction mixture (M1), in particular towards an optimum production of the desired mixture of the $(C_{4+})$, preferably the $(C_{5+})$ alkanes.

The process also comprises stage (6) comprising separating the mixture of the $(C_{4+})$, preferably $(C_{5+})$ alkanes from the reaction mixture (M1), so as to isolate and particularly to recover the liquid mixture of the $(C_{4+})$, preferably the $(C_{5+})$ alkanes. Preferably, stage (6) is performed in combination with stage (2), in particular simultaneously with stage (2). The reaction mixture (M1), as previously described in stage (1), generally comprises the mixture of the $(C_{4+})$, preferably the $(C_{5+})$ alkanes and methane, and optionally unreacted ethane, propane and where applicable butane(s). Stage (6) is preferably performed by fractionation of the reaction mixture (M1) into (a) the mixture of the $(C_{4+})$, preferably the $(C_{5+})$ alkanes, (b) the methane and optionally (c) the unreacted ethane, propane and where applicable butane(s), so as to isolate (a) said mixture of the $(C_{4+})$, preferably the $(C_{5+})$ alkanes in a liquid form, (b) the methane and optionally (c) said unreacted ethane, propane and where applicable butane(s) which are preferably returned to stage (1), in particular into the reaction zone (Z1).

Stage (6) can be performed in various ways, preferably in combination with stage (2), more particularly simultaneously with stage (2), e.g. in the fractionation zone (F1) as previously described in stage (2), either intermittently (or discontinuously) or preferably continuously. It can comprise any method or any combination of methods of fractionation for separating (a) the mixture of the $(C_{4+})$, preferably the $(C_{5+})$ alkanes, (b) methane and optionally (c) unreacted ethane, propane and where applicable butane(s). Stage (6) can comprise one or more methods of fractionation preferably chosen from the six above-mentioned types of fractionation.

Stage (6) can be performed in a fractionation zone (F3), preferably the fractionation zone (F1), which can be either separate and distinct from the reaction zone (Z1) wherein stage (1) is performed, more particularly in at least one distillation/condensation column or tower, or arranged in a part of said reaction zone (Z1), e.g. in a distillation/condensation column reactor, as described in American patent US 4,242,540.

More generally, the fractionation in stage (6) can be essentially performed by change of the physical state, preferably of the gaseous/liquid phase of the reaction mixture (M1), in particular by distillation or by condensation (or liquefaction), more particularly by partial condensation (or by partial liquefaction), e.g. in at least one distillation/condensation column or tower, or in a distillation/condensation column reactor. The fractionation in stage (6) can be performed by refrigeration or cryogenic processes, preferably by cooling of the reaction mixture (M1) to a temperature at which the mixture of the $(C_{4+})$, preferably the $(C_{5+})$ alkanes condenses, so as to condense said mixture, and to separate and to isolate it in a liquid form from the rest of the reaction mixture (M1) which can be preferably treated as in stage (2) for isolating the methane.

Generally, the fractionation in stage (6) can be also performed by molecular filtration, in particular by passing the reaction mixture (M1) through at least one semi-permeable and selective membrane, so as to arrest and to isolate the mixture of the $(C_{4+})$, preferably the $(C_{5+})$ alkanes, and to allow the rest of the reaction mixture (M1) to pass through and preferably to be treated as in stage (2) for isolating the methane.

The semi-permeable and selective membrane can be chosen from membranes especially suitable for separating the $(C_{4+})$, preferably the $(C_{5+})$ alkanes, from methane and optionally ethane, propane and where applicable butane(s), e.g. membranes preferably selected from zeolite membranes, porous alumina membranes, ceramic membranes, flowing liquid membranes and supported liquid membranes.

Generally, the fractionation in stage (6) can be also performed by adsorption, in particular by passing the reaction mixture (M1) onto at least one molecular sieve or any other adsorbent, so as to retain the mixture of the $(C_{4+})$, preferably the $(C_{5+})$ alkanes, and to allow the rest of the reaction mixture (M1) to pass through, and then by subjecting the mixture thus retained to at least one desorption step, preferably by the TSA or the PSA method, so as to isolate said mixture of the $(C_{4+})$, preferably the $(C_{5+})$ alkanes, the rest of the reaction mixture (M1) being preferably treated as in stage (2) for isolating the methane.

The adsorbents can be chosen from adsorbents especially suitable for retaining the $(C_{4+})$, preferably the $(C_{5+})$ alkanes, e.g. chosen from molecular sieves, silica gels, activated charcoals and activated carbons.

Generally, the fractionation in stage (6) can be also performed by absorption, in particular by contacting the reaction mixture (M1) with an absorption oil, e.g. in an absorption tower, so as to soak up the mixture of the $(C_{4+})$, preferably the $(C_{5+})$ alkanes, and to form an oil/alkane mixture, and then by subjecting said oil/alkane mixture, e.g. in a recovery tower, to a temperature above the boiling point of said mixture of the $(C_{4+})$, preferably the $(C_{5+})$ alkanes, but below that of the absorbing oil, so as to separate and to isolate said mixture of the $(C_{4+})$, preferably the $(C_{5+})$ alkanes from the absorbing oil, while the rest of the reaction mixture being preferably treated as in stage (2) for isolating the methane.

Generally, the fractionation in stage (6) can be also performed by a cryogenic expansion, in particular by dropping the temperature of the reaction mixture (M1) e.g. by means of external refrigerants, and then by means of an expansion turbine which rapidly expands the chilled reaction mixture (M1) and causes the temperature to drop significantly, so as to condense the mixture of the ($C_{4+}$), preferably the ($C_{5+}$) alkanes, to separate and to isolate said mixture of the ($C_{4+}$), preferably the ($C_{5+}$) alkanes in liquid form from the rest of the reaction mixture (M1) which can be preferably treated as in stage (2) for isolating the methane;

Generally, the fractionation in stage (6) can be also performed by compression, in particular by compressing the reaction mixture (M1), e.g. by means of a gas compressor, so as to condense the mixture of the ($C_{4+}$), preferably the ($C_{5+}$) alkanes, to separate and to isolate said mixture of the ($C_{4+}$), preferably the ($C_{5+}$) alkanes in liquid form from the rest of the reaction mixture (M1) which can be preferably treated as in stage (2) for isolating the methane.

Stage (6) which is preferably performed in combination with stage (2), can also comprise combining two or more successive fractionations of an identical or different type, in particular fractionations chosen from the six above-mentioned types of fractionation, in a chronological order or in a reverse order.

Thus, by means of a double or multiple fractionation, the desired mixture of the ($C_{4+}$), preferably the ($C_{5+}$) alkanes can be isolated and in particular recovered in liquid form, while the methane can be isolated and recycled into stage (3), and optionally the unreacted ethane, propane and where applicable butane(s) can be furthermore separated and isolated, and preferably recycled into stage (1).

The process of the invention has the advantage of converting ethane easily with a high yield into the mixture of the ($C_{4+}$), preferably the ($C_{5+}$) alkanes, which is capable of being easily transportable in liquid form under conditions relatively close to standard temperature and pressure conditions. It also exhibits a very high "carbon atom efficiency" which can be equal to or higher than 95%. Furthermore, it can advantageously produce great amounts of hydrogen which can be isolated and then can supply sufficient thermal and/or electrical energies for running the present process.

The following examples illustrate the present invention.

## Example 1: Preparation of a metal catalyst (C1): an organometallic tungsten hydride grafted onto alumina.

In a first stage, 2 g of an $\alpha$-alumina containing 90 wt % of alumina and 9 wt % of water, and sold by Johnson Matthey (Great Britain) were subjected to a calcination treatment under a dry air current at 500°C for 15 hours, then to a dehydroxylation treatment under an absolute pressure of $10^{-2}$ Pa, at 500°C, for 15 hours. At the end of this time, the alumina thus treated was cooled under an argon atmosphere to 25°C. In a second stage, 2 g of the alumina prepared beforehand were isolated and introduced under an argon atmosphere into a reactor fitted with an agitator at 25°C. Then, 305 mg of a precursor (Pr'), namely tungsten tris(neopentyl)neopentylidyne of the general formula (9):

$$W [ - CH_2 - C(CH_3)_3]_3 [ \equiv C - C(CH_3)_3] \qquad (9)$$

were introduced into the reactor. Then, the reactor was heated to 66°C, and the mixture thus obtained was agitated in the dry state for 4 hours. At the end of this time, the reactor was cooled to 25°C, and the solid mixture thus obtained was then washed with n-pentane at 25°C. The solid compound thus washed was vacuum dried and then isolated under an argon atmosphere, so as to obtain an organometallic tungsten compound grafted onto alumina and containing 3.9 wt % of tungsten.

In a third stage, 40 mg of the organometallic tungsten compound grafted onto alumina prepared beforehand were isolated in a reactor and subjected to a hydro-genolysis treatment, by contacting said compound with hydrogen under an absolute hydrogen pressure of 73 kPa, at 150°C, for 15 hours. At the end of this time, the reactor was cooled to 25°C, and there was obtained and isolated under an argon atmosphere a metal catalyst (C1) based on an organometallic tungsten hydride grafted onto alumina which contained 3.9 wt % of tungsten.

## Example 2: Preparation of a metal catalyst (C2): an organometallic tantalum hydride grafted onto silica.

In a first stage, 50 mg of a silica sold under the trade name "Aerosil 200"® by Degussa (Germany) were subjected to a dehydroxylation treatment under an absolute pressure of $10^{-2}$ Pa, at 700°C, for 15 hours. At the end of this time, the treated silica was cooled to 25°C.

In a second stage, 50 mg of the silica prepared beforehand were isolated and introduced into a reactor at 25°C under an argon atmosphere. A precursor (Pr) was then introduced, namely tantalum tris(neopentyl)neopentylidene, of general formula (10):

$$Ta\ [\text{-}\ CH_2 - C(CH_3)_3]_3\ [=CH - C(CH_3)_3] \qquad (10)$$

The reactor was then heated to 70°C for 2 hours, so as to sublime the precursor (Pr) onto the silica and to form an organometallic tantalum compound grafted onto the silica. At the end of this time, the surplus of unreacted precursor (Pr) was eliminated by inverse sublimation at 70°C. The reactor was then cooled to 25°C, and the organometallic tantalum compound thus grafted onto the silica was isolated under an argon atmosphere. It contained 5.5 wt % of tantalum.

In a third stage, the organometallic tantalum compound grafted onto the silica prepared beforehand was subjected to a hydrogenolysis treatment, by contacting said compound with hydrogen, under an absolute hydrogen pressure of 73 kPa, at 150°C, for 15 hours. At the end of this time, a metal catalyst (C2) based on an organometallic tantalum hydride grafted onto silica and containing 5.5 wt % of tantalum was obtained and isolated under an argon atmosphere.

**Example 3: Conversion of ethane into a liquid mixture of the (C$_{5+}$) alkanes.**

In a preliminary stage, ethane was continuously separated from a natural gas containing 10 % in mole of ethane and $C_3$ to $C_6$ alkanes. The preliminary stage comprised continuously fractionating the natural gas into (a) methane, (b) ethane and (c) the mixture of $C_3$ to $C_6$ alkanes, so as to separate, to isolate and to recover the ethane. The fractionation of the natural gas was carried out continuously by changing the physical state of the natural gas, in particular by cooling and selective liquefaction.

According to stage (1) of the process, the ethane thus isolated in preliminary stage was introduced continuously into a reactor (R1) heated to 150°C, under an absolute pressure of 2 MPa, and containing the catalyst (C1) prepared in Example 1, so as ethane was continuously contacted with the catalyst (C1). By simultaneous ethane self- and cross-metathesis reactions, there continuously formed in the reactor (R1) a reaction mixture (M1) comprising methane, a mixture of the (C$_{5+}$) alkanes and unreacted ethane, propane and butanes. The reaction mixture (M1) was withdrawn continuously out of the reactor (R1).

According to stage (2) of the process, the reaction mixture (M1) thus withdrawn from reactor (R1) was introduced continuously into a fractionation zone (F1) wherein methane was separated and isolated from the reaction mixture (M1). The fractionation of the reaction mixture (M1) was carried out continuously by changing the physical state of said reaction mixture, in particular by cooling and selective liquefaction, so as to isolate the methane in a gaseous form from the rest of said reaction mixture separated in a liquid form. The methane thus isolated was withdrawn continuously out of the fractionation zone (F1), while the rest of the reaction mixture was recycled continuously into the reactor (R1), where the simultaneous ethane self- and cross-metathesis reactions were continued developing towards the production of the mixture of the (C$_{5+}$) alkanes.

According to stage (3) of the process, the methane isolated in stage (2) was introduced continuously into a reactor (R2) heated to 475°C and under an absolute pressure of 5 MPa, the reactor containing the catalyst (C2) prepared in Example 2, so as methane was continuously contacted with the catalyst (C2). By a non-oxidative methane coupling reaction, there continuously formed in the reactor (R2) a gaseous mixture (M2) comprising ethane, hydrogen and unreacted methane. The gaseous mixture (M2) was withdrawn continuously out of the reactor (R2).

According to stage (4) of the process, the gaseous mixture (M2) thus withdrawn from reactor (R2) was introduced continuously into a fractionation zone (F2) wherein ethane, hydrogen and the unreacted methane were separated and isolated from the gaseous mixture (M2). The fractionation of the mixture (M2) was continuously carried out by the change of the physical state of said mixture (M2), in particular by cooling and selective liquefaction. The unreacted methane thus isolated was recycled continuously into reactor (R2) where the non-oxidative methane coupling reaction ethane was continued.

According to stage (5) of the process, the ethane isolated in stage (4) was continuously recycled into stage (1), in particular by a continuous introduction into the reactor (R1), especially for going on with the simultaneous ethane self- and cross-metathesis reactions and continuing forming the reaction mixture (M1) and particularly the mixture of the (C$_{5+}$) alkanes. Simultaneously, a part of the hydrogen isolated in stage (4) was continuously introduced into the reactor (R1) especially for maintaining the catalyst (C1) with a high activity.

According to stage (6) of the process carried out in combination and simultaneously with stage (2), the mixture of the (C$_{5+}$) alkanes was continuously separated and isolated from the reaction mixture (M1) in a fractionation zone (F3) arranged with the fractionation zone (F1), comprising changing the physical state of said reaction mixture (M1), in particular by cooling and selective liquefaction. The mixture of the (C$_{5+}$) alkanes was continuously withdrawn from the fractionation zone (F3) and recovered in the form of a liquid mixture, essentially comprising pentanes, hexanes, heptanes and octanes.

**<u>Example 4:</u> Conversion of ethane into a liquid mixture of the (C$_{5+}$) alkanes.**

The conversion was exactly identical to the conversion carried out in Example 3, apart from the fact that the preliminary stage comprised continuously fractionating the natural gas into (a) methane and (b) ethane in a mixture with the C$_3$ to C$_6$ alkanes, ethane being in a major molar proportion in said mixture, and apart from the fact that in stage (1), ethane in said mixture with the C$_3$ to C$_6$ alkanes thus isolated in the preliminary stage was continuously introduced into the reactor (R1) instead of ethane only.

Thus, in stage (6), a mixture of the (C$_{5+}$) alkanes was continuously withdrawn from the fractionation zone (F3) and recovered in the form of a liquid mixture, essentially comprising pentanes, hexanes, heptanes, octanes and nonanes.

**Claims**

1. Process for converting ethane into a liquid mixture of (C$_{4+}$) alkanes having 4 carbon atoms and more, preferably (C$_{5+}$) alkanes having 5 carbon atoms and more, **characterised in that** it comprises:

   - a stage (1) comprising simultaneous ethane self- and cross-metathesis reactions carried out by contacting ethane with a metal catalyst (C1) capable of producing, in contact with alkane, reactions involving the splitting and recombining of carbon-carbon and/or carbon-hydrogen and/or carbon-metal bonds, so as to form a reaction mixture (M1) comprising methane and a mixture of the (C$_{4+}$), preferably the (C$_{5+}$) alkanes,
   - a stage (2) comprising separating methane from the reaction mixture (M1), so as to isolate the methane,
   - a stage (3) comprising a non-oxidative methane coupling reaction carried out by contacting the methane thus isolated with a metal catalyst (C2) capable of producing, in contact with alkane, reactions involving the splitting and recombining of carbon-carbon and/or carbon-hydrogen and/or carbon-metal bonds, said catalyst being identical to or different from the catalyst (C1), so as to form a mixture (M2) comprising ethane and hydrogen,
   - optionally a stage (4) comprising separating the ethane from the mixture (M2), so as to isolate the ethane,
   - a stage (5) comprising recycling the ethane thus isolated or the mixture (M2) into stage (1) for simultaneous ethane self- and cross-metathesis reactions and for continuing forming said reaction mixture (M1), and
   - a stage (6) comprising separating the mixture of the (C$_{4+}$), preferably the (C$_{5+}$) alkanes from the reaction mixture (M1), so as to isolate the liquid mixture of the (C$_{4+}$), preferably the (C$_{5+}$) alkanes, stage (6) being preferably performed in combination with stage (2).

2. Process according to claim 1, **characterised in that** ethane used in stage (1) is selected from ethane separated from natural gas, or from ethane resulting from a process manufacturing ethane as the main product or as a by-product.

3. Process according to claim 1 or 2, **characterised in that**, in the contacting of stage (1), ethane is used alone or in the form of a mixture of ethane, preferably in a major molar proportion, with one or more other alkane(s), in particular selected from (C$_{3+}$) alkanes having at least 3 carbon atoms, more particularly from the (C$_{3+}$) alkanes of the natural gas.

4. Process according to claim 3, **characterised in that** ethane used in stage (1) is previously separated from natural gas in a preliminary stage comprising fractionating natural gas into (a) methane, (b) ethane and (c) a mixture of the (C$_{3+}$) alkanes having at least 3 carbon atoms of the natural gas, or into (a) methane and (b) ethane in a mixture with said mixture of the (C$_{3+}$) alkanes, so as to separate and to isolate the ethane optionally in a mixture with said mixture of the (C$_{3+}$) alkanes.

5. Process according to any one of claims 1 to 4, **characterised in that** the catalysts (C1) and (C2) are chosen from metal catalysts capable of alkane metathesis, preferably from supported metal clusters and from metal hydrides, organometallic compounds and organometallic hydrides, more particularly supported on, preferably grafted onto, a solid support.

6. Process according to claim 5, **characterised in that** the supported metal clusters comprise one or more metals of Groups 4, 5 and/or 6 of the Periodic Table of the Elements, preferably tantalum.

7. Process according to claim 5 or 6, **characterised in that** the supported metal clusters comprise a solid support and a metal cluster, preferably bonding between the metals of the metal cluster, and bonding between the metal cluster and the solid support.

8. Process according to claim 7, **characterised in that** the metal atom of the metal cluster is bonded to at least one other metal of the metal cluster, preferably to six or fewer metal atoms of the metal cluster.

9. Process according to any one of claims 5 to 8, **characterised in that** the supported metal clusters comprise a solid support chosen from a metal oxide, a molecular sieve, a zeolite, a metal phosphate and a material comprising a metal and oxygen, preferably silica.

10. Process according to claim 5, **characterised in that** the catalysts chosen from metal hydrides, organometallic compounds and organometallic hydrides, comprise at least one metal chosen from lanthanides, actinides and metals of Groups 2 to 12, preferably transition metals of Groups 3 to 12 of the Periodic Table of the Elements.

11. Process according to claim 10, **characterised in that** the catalysts comprise at least one metal chosen from scandium, yttrium, lanthanum, titanium, zirconium, hafnium, vanadium, niobium, tantalum, chromium, molybdenum, tungsten, rhenium, ruthenium, osmium, nickel, iridium, palladium, platinum, cerium and neodymium, preferably from yttrium, titanium, zirconium, hafnium, vanadium, niobium, tantalum, chromium, molybdenum, tungsten, rhenium, ruthenium and platinum, and more particularly from niobium, tantalum, molybdenum, tungsten and rhenium.

12. Process according to claim 10 or 11, **characterised in that** the catalysts comprise one or more ligands comprising at least one oxygen atom and/or at least one nitrogen atom, preferably chosen from oxo, alkoxo, aryloxo, aralkyloxo, nitrido, imido and amido ligands.

13. Process according to any one of claims 10 to 12, **characterised in that** the catalysts are supported on, or grafted onto, a solid support chosen from metal or refractory oxides, sulfides, carbides, nitrides and salts, and from carbon, mesoporous materials, organic/inorganic hybrid materials, metals and molecular sieves.

14. Process according to any one of claims 1 to 13, **characterised in that** stage (1) comprises contacting the catalyst (C1) with ethane in a mixture with one or more other alkanes preferably selected from $(C_{3+})$ alkanes having at least 3 carbon atoms, in particular the $(C_{3+})$ alkanes of natural gas.

15. Process according to any one of claims 1 to 14, **characterised in that** stage (1) is performed by adding ethane optionally in a mixture with one or more other alkanes to the catalyst (C1), or by adding the catalyst (C1) to ethane optionally in a mixture with one or more other alkanes, or by simultaneously adding the catalyst (C1) and ethane optionally in a mixture with one or more other alkanes to a reaction zone (Z1).

16. Process according to any one of claims 1 to 15, **characterised in that** stage (1) is performed at a temperature chosen in a range of from 20 to 400°C, preferably from 50 to 350°C, in particular from 70 to 300°C, especially from 100 to 250°C.

17. Process according to any one of claims 1 to 16, **characterised in that** stage (1) is performed under an absolute total pressure chosen in a range of from 0.1 to 50 MPa, preferably from 0.1 to 30 MPa, in particular from 0.1 to 20 MPa.

18. Process according to any one of claims 1 to 17, **characterised in that** stage (1) is performed in the presence of hydrogen, in particular under a partial pressure of hydrogen chosen in a range of from 0.0001 to 10 MPa, preferably from 0.001 to 1 MPa.

19. Process according to any one of claims 1 to 18, **characterised in that** stage (1) is performed discontinuously or preferably continuously.

20. Process according to any one of claims 1 to 19, **characterised in that** stage (1) is performed in a reaction zone (Z1) which is either distinct and separate from a fractionation zone (F1) wherein stage (2) is performed, or arranged in a part of said fractionation zone (F1), preferably in a distillation/condensation column reactor.

21. Process according to any one of claims 1 to 20, **characterised in that** stage (1) is performed in a reaction zone (Z1) comprising at least one reactor chosen from static reactors, recycling reactors and dynamic reactors.

22. Process according to any one of claims 1 to 21, **characterised in that** stage (1) is performed in a reaction zone (Z1) comprising at least one reactor chosen from tubular or multiple-tubular reactors, distillation/condensation column reactors, slurry reactors, fluidised bed reactors, mechanically agitated bed reactors, fluidised and mechanically agitated bed reactors, fixed bed reactors, moving bed reactors and circulating bed reactors.

**23.** Process according to any one of claims 1 to 22, **characterised in that** stage (1) is performed (i) by continuously introducing ethane into stage (1), in particular into a reaction zone (Z1) containing the catalyst (C1), so as to form continuously the reaction mixture (M1), and (ii) by continuously withdrawing at least a part of the reaction mixture (M1) out of stage (1), in particular out of the reaction zone (Z1), so as to subject said part of the reaction mixture to stage (2) and to stage (6) preferably for separating the mixture of the $(C_{4+})$, preferably the $(C_{5+})$ alkanes from the reaction mixture (M1) and isolating the liquid mixture of the $(C_{4+})$, preferably the $(C_{5+})$ alkanes from the rest of the reaction mixture which is preferably returned to stage (1), in particular into the reaction zone (Z1).

**24.** Process according to any one of claims 1 to 23, **characterised in that** stage (2) is performed in a fractionation zone (F1) by fractionating the reaction mixture (M1) into (a) methane and (b) the rest of the reaction mixture preferably comprising the mixture of the $(C_{4+})$, preferably the $(C_{5+})$ alkanes and optionally the unreacted ethane, propane and where applicable butane(s), so as to isolate (a) the methane and (b) said rest of the reaction mixture which is preferably returned to stage (1).

**25.** Process according to any one of claims 1 to 24, **characterised in that** stage (2) is performed discontinuously or preferably continuously.

**26.** Process according to any one of claims 1 to 25, **characterised in that** stage (2) comprises one or more methods of fractionation of the mixture (M1) chosen from (i) fractionation by change of physical state, preferably of gaseous/ liquid phase, particularly by distillation or by condensation, more particularly by partial condensation, preferably by means of distillation/condensation column or column-reactor, (ii) fractionation by molecular filtration, preferably by means of semi-permeable and selective membrane, (iii) fractionation by adsorption, preferably by means of molecular sieve or any other adsorbent, (iv) fractionation by absorption, preferably by means of absorbing oil, (v) fractionation by cryogenic expansion, preferably by means of expansion turbine, and (vi) fractionation by compression, preferably by means of gas compressor.

**27.** Process according to any one of claims 1 to 26, **characterised in that** stage (2) is performed in a fractionation zone (F1) which is either separate and distinct from a reaction zone (Z1) wherein stage (1) is performed, more particularly comprising at least one distillation/condensation column or tower, or arranged in a part of said reaction zone (Z1), preferably in a distillation/condensation column reactor.

**28.** Process according to any one of claims 1 to 27, **characterised in that** stage (3) is performed at a temperature chosen in a range of from 150 to 700°C, preferably from 200 to 650°C, more particularly from 300 to 600°C.

**29.** Process according to any one of claims 1 to 28, **characterised in that** stage (3) is performed under an absolute total pressure chosen in a range of from 0.1 to 50 MPa, preferably from 0.1 to 30 MPa, more particularly from 0.1 to 20 MPa.

**30.** Process according to any one of claims 1 to 29, **characterised in that** stage (3) is performed discontinuously or preferably continuously.

**31.** Process according to any one of claims 1 to 30, **characterised in that** stage (3) comprises adding the methane to the catalyst (C2), or adding the catalyst (C2) to the methane, or simultaneously adding the methane and the catalyst (C2) to a reaction zone (Z2).

**32.** Process according to any one of claims 1 to 31, **characterised in that** stage (3) is performed in a reaction zone (Z2) which is either distinct and-separate from fractionation zones (F1) and (F2) wherein stages (2) and (4) are respectively performed, or arranged in a part of said fractionation zone (F1) or preferably (F2), in particular in a distillation/condensation column reactor.

**33.** Process according to any one of claims 1 to 32, **characterised in that** stage (3) is performed in a reaction zone (Z2) comprising at least one reactor chosen from static reactors, recycling reactors and dynamic reactors.

**34.** Process according to any one of claims 1 to 33, **characterised in that** stage (3) is performed in a reaction zone (Z2) comprising at least one reactor chosen from tubular or multiple-tubular reactors, distillation/condensation column reactors, slurry reactors, fluidised bed reactors, mechanically agitated bed reactors, fluidised and mechanically agitated bed reactors,, fixed bed reactors, moving bed reactors and circulating bed reactors.

35. Process according to any one of claims 1 to 34, **characterized in that** stage (3) is performed (i) by continuously introducing methane into stage (3), in particular into a reaction zone (Z2) containing the catalyst (C2), so as to form continuously the mixture (M2), and (ii) by continuously withdrawing at least one part of the mixture (M2) out of stage (3), in particular out of the reaction zone (Z2), so as to submit said part of the mixture (M2) to stage (4) for separating and isolating ethane from the rest of the mixture (M2), and recycling the ethane thus separated into stage (1), and preferably returning said rest of the mixture, particularly the unreacted methane into stage (3), in particular into the reaction zone (Z2).

36. Process according to any one of claims 1 to 35, **characterised in that** stage (4) comprises separating ethane and optionally hydrogen from the mixture (M2), so as to isolate the ethane, optionally the hydrogen and optionally the unreacted methane, or to isolate the ethane in a mixture with hydrogen.

37. Process according to any one of claims 1 to 36, **characterised in that** stage (4) is performed discontinuously or preferably continuously.

38. Process according to any one of claims 1 to 37, **characterised in that** stage (4) comprises one or more methods of fractionation preferably selected from (i) fractionation by change of physical state, in particular by change of gaseous/liquid phase, more particularly by distillation or by condensation, especially by partial condensation, preferably by means of distillation/condensation column or column-reactor, (ii) fractionation by molecular filtration, preferably by means of semi-permeable and selective membrane, (iii) fractionation by adsorption, preferably by means of molecular sieve or any other adsorbent, (iv) fractionation by absorption, preferably by means of absorbing oil, (v) fractionation by cryogenic expansion, preferably by means of expansion turbine, and (vi) fractionation by compression, preferably by means of gas compressor.

39. Process according to any one of claims 1 to 38, **characterised in that** stage (4) is performed in a fractionation zone (F2) which is either separate and distinct from a reaction zone (Z2) wherein stage (3) is performed, preferably comprising at least one distillation/condensation column or tower, or arranged in a part of said reaction zone (Z2), preferably in a distillation/condensation column reactor.

40. Process according to any one of claims 1 to 39, **characterised in that** stage (4) comprises separating and isolating from the mixture (M2) the unreacted methane which is preferably recycled into stage (3).

41. Process according to any one of claims 1 to 40, **characterised in that** stage (4) comprises separating and isolating from the mixture (M2) the hydrogen which is preferably used in one or more applications or stages of the process, in particular (i) as an agent for activation or regeneration of the catalysts (C1) and/or (C2), or (ii) as a fuel for producing thermal and/or electrical energies, in particular as a fuel in hydrogen fuel cells for producing electrical energies, preferably employed in the process, or (iii) as a fuel for automobile, or (iv) as a reagent in a chemical, petrochemical or refinery plant.

42. Process according to any one of claims 1 to 41, **characterised in that** stage (6) is performed by fractionation of the reaction mixture (M1) into (a) the mixture of the $(C_{4+})$, preferably the $(C_{5+})$ alkanes, (b) the methane and optionally (c) the unreacted ethane, propane and where applicable butane(s), so as to isolate (a) said mixture of the $(C_{4+})$, preferably the $(C_5+)$ alkanes in a liquid form, (b) the methane and optionally (c) said unreacted ethane, propane and where applicable butane(s) which are preferably returned to stage (1).

43. Process according to any one of claims 1 to 42, **characterised in that** stage (6) is performed in combination with stage (2), preferably simultaneously with stage (2), either discontinuously or preferably continuously.

44. Process according to any one of claims 1 to 43, **characterised in that** stage (6) is performed in a fractionation zone (F3), preferably the fractionation zone (F1) wherein stage (2) is performed, which is either separate and distinct from a reaction zone (Z1) wherein stage (1) is performed, preferably in at least one distillation/condensation column or tower, or arranged in a part of said reaction zone (Z1), preferably in a distillation/condensation column reactor.

45. Process according to any one of claims 1 to 44, **characterised in that** stage (6) comprises one or more methods selected from (i) fractionation by change of physical state, preferably by change of gaseous/liquid phase, in particular by distillation or by condensation, more particularly by partial condensation, preferably by means of distillation/condensation column or column-reactor, (ii) fractionation by molecular filtration, preferably by means of semi-permeable and selective membrane, (iii) fractionation by adsorption, preferably by means of molecular sieve or any other adsorbent, (iv) fractionation by absorption, preferably by means of absorbing oil, (v) fractionation by cryogenic expansion, preferably by means of expansion turbine, and (vi) fractionation by compression, preferably by means of gas compressor.

**European Patent Office**

EUROPEAN SEARCH REPORT

Application Number

EP 07 25 2370

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| D,X | WO 98/02244 A (CENTRE NAT RECH SCIENT [FR]; VIDAL VERONIQUE [FR]; THEOLIER ALBERT [FR] 22 January 1998 (1998-01-22) * claims; example 3 * ----- | 1-45 | INV. C07C6/10 C07C9/10 C07C9/14 C10G50/00 C07C2/76 C07C9/06 |
| X | WO 03/104172 A (BP CHEM INT LTD [GB]; COPERET CHRISTOPHE [FR]; DOBSON IAN [GB]; MAUNDE) 18 December 2003 (2003-12-18) * figure 4 * * page 3, line 32 - page 4, line 2 * * page 5, line 21 * * page 5, line 30 * * page 5, line 33 - page 6, line 19 * * page 28, line 14 - page 32, line 19 * ----- | 1-45 | |
| D,A | WO 03/104171 A (BP CHEM INT LTD [GB]; BASSET JEAN-MARIE [FR]; BRES PHILIPPE [FR]; COPE) 18 December 2003 (2003-12-18) * claims; examples * ----- | 1-45 | |

TECHNICAL FIELDS SEARCHED (IPC)

C07C
C10G

The present search report has been drawn up for all claims

| Place of search | Date of completion of the search | Examiner |
|---|---|---|
| Munich | 21 November 2007 | Seufert, Gudrun |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

..................................................................
& : member of the same patent family, corresponding
   document

EPO FORM 1503 03.82 (P04C01)

**ANNEX TO THE EUROPEAN SEARCH REPORT
ON EUROPEAN PATENT APPLICATION NO.**

EP 07 25 2370

This annex lists the patent family members relating to the patent documents cited in the above-mentioned European search report.
The members are as contained in the European Patent Office EDP file on
The European Patent Office is in no way liable for these particulars which are merely given for the purpose of information.

21-11-2007

| Patent document cited in search report | | | Publication date | Patent family member(s) | | | Publication date |
|---|---|---|---|---|---|---|---|
| WO 9802244 | A | | 22-01-1998 | AT | 190522 | T | 15-04-2000 |
| | | | | DE | 69701462 | D1 | 20-04-2000 |
| | | | | DE | 69701462 | T2 | 27-07-2000 |
| | | | | DK | 910469 | T3 | 21-08-2000 |
| | | | | EP | 0910469 | A1 | 28-04-1999 |
| | | | | ES | 2146109 | T3 | 16-07-2000 |
| | | | | FR | 2750894 | A1 | 16-01-1998 |
| | | | | GR | 3033371 | T3 | 29-09-2000 |
| | | | | JP | 2000514446 | T | 31-10-2000 |
| | | | | PT | 910469 | T | 31-08-2000 |
| | | | | US | 6229060 | B1 | 08-05-2001 |
| WO 03104172 | A | | 18-12-2003 | AU | 2003232358 | A1 | 22-12-2003 |
| | | | | FR | 2840606 | A1 | 12-12-2003 |
| WO 03104171 | A | | 18-12-2003 | AU | 2003232934 | A1 | 22-12-2003 |
| | | | | CA | 2488758 | A1 | 18-12-2003 |
| | | | | CN | 1659120 | A | 24-08-2005 |
| | | | | EP | 1511703 | A1 | 09-03-2005 |
| | | | | FR | 2840607 | A1 | 12-12-2003 |
| | | | | US | 2005272966 | A1 | 08-12-2005 |

EPO FORM P0459

For more details about this annex : see Official Journal of the European Patent Office, No. 12/82

**EP 2 014 635 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9802244 A
- WO 0104077 A
- WO 03066552 A
- WO 03104171 A
- WO 2006060692 A
- WO 03061823 A
- WO 2004089541 A
- US 4242530 A
- US 4242540 A

**Non-patent literature cited in the description**

- IUPAC. CRC Press, Inc, 1991
- CRC Handbook of Chemistry and Physics. 1995